# FASCICULE DE BREVET EUROPEEN

(11) **EP 4 448 618 B1**
(45) Date de publication et mention de la délivrance du brevet: **29.10.2025**
(21) Numéro de dépôt: 22836124.2
(22) Date de dépôt: 13.12.2022
(51) Int. Cl.: C08G 65/42, C07D 487/22, C12H 1/14

(54) **PROCÉDÉ DE SÉQUESTRATION DE MÉTHIONINE CONTENUE DANS UN LIQUIDE ET MATÉRIAU CONVENANT À LA MISE EN OEUVRE D'UN TEL PROCÉDÉ**
VERFAHREN ZUR SEQUESTRIERUNG VON METHIONIN IN EINER FLÜSSIGKEIT UND ZUR IMPLEMENTIERUNG SOLCH EINES VERFAHRENS GEEIGNETES MATERIAL
METHOD FOR SEQUESTERING METHIONINE CONTAINED IN A LIQUID AND MATERIAL SUITABLE FOR IMPLEMENTING SUCH A METHOD

(30) Priorité: 16.12.2021 FR 2113725
(43) Date de publication de la demande: 23.10.2024
(73) Titulaire: Université de Bordeaux, 33000 Bordeaux (FR); Institut Polytechnique de Bordeaux, 33402 Talence Cedex (FR); Centre National de la Recherche Scientifique, 75016 Paris (FR)
(72) Inventeur: BASSANI, Dario, 33600 Pessac (FR); FURET, Amaury, 33000 Bordeaux (FR); FINDLAY, James, Dunedin, 9024 (NZ)
(74) Mandataire: Ipside
(86) Numéro de dépôt international: PCT/EP2022/085685
(87) Numéro de publication internationale: WO 2023/110915

(56) Documents cités:
- FRACASSETTI DANIELA ET AL: "Light-induced reactions of methionine and riboflavin in model wine: Effects of hydrolysable tannins and sulfur dioxide", FOOD CHEMISTRY, ELSEVIER LTD, NL, vol. 298, 6 June 2019 (2019-06-06), XP085742541, ISSN: 0308-8146, [retrieved on 20190606], DOI: 10.1016/J.FOODCHEM.2019.124952
- LANZILOTTO ANGELO ET AL: "Porphyrin-polymer nanocompartments: singlet oxygen generation and antimicrobial activity", JBIC. JOURNAL OF BIOLOGICAL INORGANIC CHEMISTRY, SPRINGER, DE, vol. 23, no. 1, 7 December 2017 (2017-12-07), pages 109 - 122, XP036400296, ISSN: 0949-8257, [retrieved on 20171207], DOI: 10.1007/S00775-017-1514-8

## Description

La présente invention s'inscrit dans le domaine du traitement des liquides, notamment des boissons destinées à la consommation humaine.

Plus particulièrement, la présente invention concerne un procédé de séquestration de molécules de méthionine contenues dans un milieu liquide. L'invention concerne également un procédé plus global de traitement d'un produit liquide alimentaire pour y diminuer le risque d'apparition d'un goût de lumière, ainsi qu'un procédé de détection de la présence de méthionine dans un liquide. L'invention concerne en outre un matériau polymère particulier convenant à la mise en œuvre de tels procédés, capable de séquestrer la méthionine.

### CONTEXTE DE L'INVENTION

On entend dans la présente description, par le terme « vin », aussi bien les vins tranquilles que les vins effervescents.

Les vins sont des produits consommés dans le monde entier, et une attention particulière est accordée à leur qualité. De nombreux défauts sont susceptibles d'y apparaitre, que ce soit au cours de leur élaboration ou lors de leur stockage. La déviation aromatique la plus connue du grand public est le goût de bouchon, qui se manifeste lors de l'ouverture de la bouteille. Un autre défaut tout aussi délétère, qualifié de « goût de lumière », peut se développer suite à l'exposition du vin à la lumière. Cette altération organoleptique entraine la formation dans le vin d'un goût rappelant le choux cuit ou d'une odeur de laine mouillée. Ce défaut peut également être présent dans les produits issus de l'industrie brassicole ou de l'industrie des produits laitiers.

Dans les vins, le goût de lumière est formé suite à la réaction de photooxydation des acides aminés soufrés, qui y sont produits lors de la fermentation alcoolique, par un photosensibilisateur particulier, qui y est présent naturellement : la riboflavine. Lorsque la riboflavine absorbe la lumière, à une longueur d'onde entre 300 et 500 nm, un état excité singulet est formé. Cet état est majoritairement transformé en un état triplet par un croisement inter-système et il peut alors oxyder, par transfert d'électron, les acides aminés soufrés, et tout particulièrement la méthionine. Ces acides aminés sont ainsi dégradés en produits soufrés volatiles tels que le méthanethiol, le diméthyldisulfure et le diméthyltrisulfure. Ces espèces soufrées ont des seuils de perception très bas, et des concentrations de l'ordre du micromolaire entrainent la formation d'un goût de lumière gustativement détectable par les consommateurs. Le produit peut développer ce défaut très rapidement. Par exemple, l'exposition d'une flûte de vin effervescent à la lumière du soleil d'été pendant 15 minutes peut suffire à y former le goût de lumière. Au niveau gustatif, le produit, et c'est particulièrement le cas pour les vins blancs et rosés et pour les vins effervescents, développe une amertume prononcée.

Dans les produits laitiers, le goût de lumière est également principalement formé suite à la dégradation d'acides aminés soufrés, en particulier de la méthionine, en conséquence de sa réaction avec la riboflavine à l'état triplet. Cette réaction conduit à la formation de composés soufrés volatiles malodorants tels que le méthanethiol et le diméthyldisulfure, responsables de l'apparition du goût de lumière.

Pour éviter l'apparition du goût de lumière dans ces produits liquides, il est nécessaire de les maintenir en permanence à l'abri de la lumière, depuis leur conception jusqu'à leur consommation. Ceci s'avère dans la pratique difficilement réalisable. En particulier, les verres opaques mis en œuvre pour le conditionnement des vins, notamment des vins effervescents, soit ne présentent pas des propriétés filtrantes de la lumière suffisamment bonnes pour prévenir efficacement l'apparition du goût de lumière, soit sont très coûteux à mettre en œuvre dans le contexte d'une fabrication industrielle. En outre, l'utilisation d'emballages très opaques, qui cachent le produit, est dommageable pour la vente.

Afin de limiter le risque d'apparition du goût de lumière dans les vins, il a été proposé par l'art antérieur d'y réduire la concentration de riboflavine par différentes approches œnologiques, plus particulièrement soit en limitant sa libération par les levures lors du procédé d'élaboration du vin, par exemple par mise en œuvre, pour la fermentation alcoolique, de souches de *Saccharomyces* produisant peu de riboflavine, soit en extrayant cette dernière du vin au moyen de différents adsorbants inorganiques, tels que le charbon actif (Fracassetti et al., Australian Journal of Grape and Wine Research 23, 329-333, 2017). La diminution de la concentration de riboflavine dans les vins a cependant pour conséquence d'en atténuer la coloration. En outre, le traitement du vin avec des adsorbants tels que le charbon actif peut également entrainer une diminution de la concentration des autres composés aromatiques du vin, notamment des polyphénols. Un tel traitement entrainerait donc une modification du profil aromatique du vin et la détérioration de ses propriétés organoleptiques. L'utilisation de tanins hydrolysables a également été utilisé pour protéger les vins blancs contre l'apparition du goût de lumière (Fracassetti et al., Food Chemistry 298, 1-8, 124952, 2019).

Ainsi, à l'heure actuelle, mis à part l'utilisation d'un emballage très opaque, peu adapté à de nombreuses boissons destinées à la consommation humaine, il n'existe aucune solution satisfaisante permettant de protéger les liquides alimentaires, en particulier les vins et produits laitiers, vis-à-vis de l'apparition du goût de lumière, ceci sans en modifier le profil sensoriel.

En cherchant une solution pour prévenir l'apparition du goût de lumière due à la formation de composés soufrés volatils lors de l'exposition à la lumière de produits liquides contenant des acides aminés soufrés et de la riboflavine, les présents inventeurs ont recherché à extraire de ces produits, non pas la riboflavine, comme cela a été proposé par l'art antérieur, mais la méthionine, acide aminé soufré principalement à l'origine, avec la riboflavine, de la formation de ces composés soufrés volatils délétères.

Les présents inventeurs ont découvert que des matériaux polymères particuliers permettent de séquestrer efficacement les molécules de méthionine contenues dans les milieux liquides, et de les en éliminer, et ils ont constaté que l'apparition du goût de lumière est alors réduite de manière significative lorsque ces milieux liquides sont ensuite exposés à la lumière dans la plage de longueur d'onde susceptible d'exciter la riboflavine. Cette inhibition de l'apparition du goût de lumière est en outre obtenue sans modifier les propriétés organoleptiques du milieu liquide, ce qui s'avère tout à fait avantageux dans le contexte des boissons destinées à la consommation humaine.

Ainsi, la présente invention vise à remédier aux inconvénients des solutions proposées par l'art antérieur pour prévenir l'apparition du goût de lumière dans les boissons, notamment aux inconvénients exposés ci-avant, en proposant un procédé qui permette de prévenir efficacement une telle apparition, sans pour autant modifier le profil sensoriel de la boisson. Plus généralement, la présente invention vise à proposer un procédé permettant de séquestrer de manière efficace des molécules de méthionine contenues dans un milieu liquide.

Des objectifs supplémentaires de l'invention sont que ce procédé soit simple à mettre en œuvre, et à faible coût.

### RESUME DE L'INVENTION

Selon un premier aspect, il est ainsi proposé selon la présente invention un procédé de séquestration de molécules de méthionine contenues dans un milieu liquide, comprenant la mise en contact de ce milieu liquide avec un matériau polymère solide comprenant des unités monomères à base de porphyrine contenant du cuivre.

Dans des modes de mise en œuvre préférés de l'invention, le matériau polymère solide contient au moins un groupement hydrophile, de préférence au moins un groupement ionique, préférentiellement au moins un groupement anionique, de préférence encore au moins un groupement ion sulfonate ou un de ses sels. Ce groupement peut en particulier être porté par au moins une desdites unités monomères à base de porphyrine contenant du cuivre du polymère, de préférence dans chacune de ces unités monomères.

Un polymère particulièrement adapté à la mise en œuvre de ce procédé répond à la formule générale (V) : 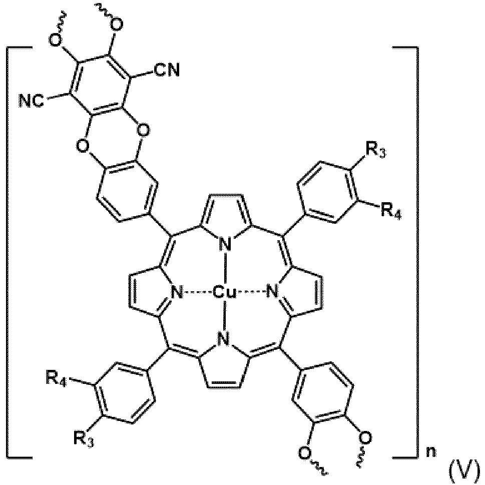 dans laquelle :
R₃ et R₄, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique,
et n est un nombre entier supérieur à 2, et de préférence inférieure à 1000.

Un autre polymère convenant particulièrement à la mise en œuvre de ce procédé est tel que susceptible d'être obtenu par un procédé de synthèse comprenant des étapes de :
a/ préparation d'un dérivé de porphyrine de formule (VI) : 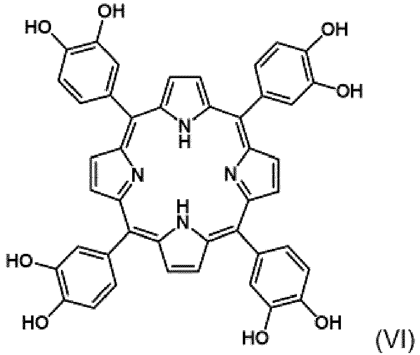
b/ polycondensation du dérivé de porphyrine de formule (VI) avec un composé de formule (VII) : 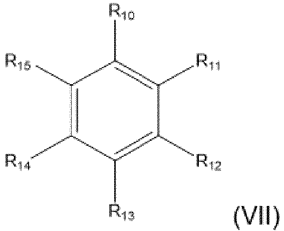 dans laquelle :
   R₁₀ et R₁₃ représentent chacun un groupement carbonitrile -CN,
   et R₁₁, R₁₂, R₁₄ et R₁₅, identiques ou différents, représentent chacun un atome d'halogène, notamment un atome de fluor,
   en présence d'un composé de formule (VIII) : 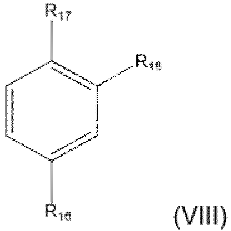 dans laquelle :
      R₁₆ représente un groupement hydroxyle, amine primaire ou secondaire ou thiol, et R₁₇ et R₁₈, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique,
      de sorte à former un polymère dont des unités monomères contiennent un noyau porphyrine, et
c/ complexation des noyaux porphyrine du polymère obtenu à l'étape b/ avec du cuivre.

Selon un autre aspect, la présente invention concerne un procédé de traitement d'un produit liquide alimentaire, notamment d'une boisson destinée à la consommation humaine, pour y diminuer, voire supprimer, le risque d'apparition de goût de lumière, ce procédé comprenant la mise en œuvre, sur ce produit liquide alimentaire, d'un procédé de séquestration de molécules de méthionine contenues dans un milieu liquide selon l'invention, puis, après un temps de contact d'au moins 30 minutes, de préférence d'au moins 2 heures, notamment compris entre 2 heures et 24 mois, par exemple entre 4 heures et 24 mois, la séparation du matériau polymère et du produit liquide alimentaire.

Ce produit alimentaire peut notamment être choisi parmi les vins, notamment les vins blancs, les vins rosés et les vins effervescents, les produits laitiers et les produits brassicoles, et plus généralement tous les produits alimentaires liquides contenant de la méthionine susceptible d'être dégradée.

Un autre aspect de l'invention est un procédé de détection de la présence de méthionine dans un liquide, qui comprend la mise en œuvre, sur un échantillon de ce liquide, d'un procédé de séquestration de molécules de méthionine contenues dans un milieu liquide selon l'invention, puis, après un temps de contact d'au moins 30 minutes, la séparation du matériau polymère et du liquide et l'analyse du matériau polymère ainsi récupéré pour la présence de méthionine.

L'invention concerne également un matériau polymère solide poreux particulièrement adapté à la mise en œuvre des procédés selon l'invention. Ce matériau polymère est tel que susceptible d'être obtenu par un procédé de synthèse comprenant des étapes de :
a/ préparation d'un dérivé de porphyrine de formule (VI) : 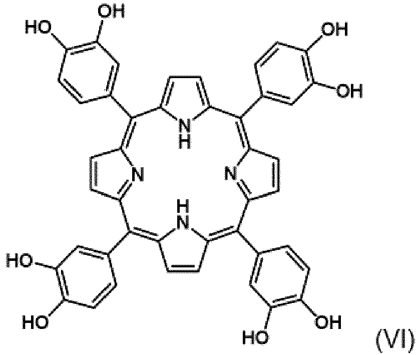
b/ polycondensation du dérivé de porphyrine de formule (VI) avec un composé de formule (VII) : 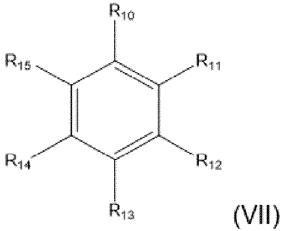 dans laquelle :
   R₁₀ et R₁₃ représentent chacun un groupement carbonitrile -CN,
   et R₁₁, R₁₂, R₁₄ et R₁₅, identiques ou différents, représentent chacun un atome d'halogène, notamment un atome de fluor,
   en présence d'un composé de formule (VIII) : 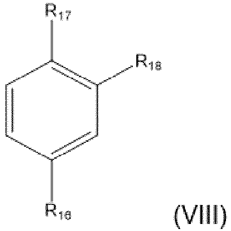 dans laquelle :
      R₁₆ représente un groupement hydroxyle, amine primaire ou secondaire ou thiol, et R₁₇ et R₁₈, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique,
      de sorte à former un polymère dont des unités monomères sont à base de noyau porphyrine, et
c/ complexation des noyaux porphyrine du polymère obtenu à l'étape b/ avec du cuivre.

### BREVE DESCRIPTION DES FIGURES

La figure 1 représente un exemple de schéma réactionnel pour la synthèse d'un polymère linéaire pouvant être mis en œuvre conformément à l'invention.
La figure 2 représente un exemple de schéma réactionnel pour la synthèse d'un polymère réticulé pouvant être mis en œuvre conformément à l'invention.
La figure 3 montre le spectre RMN ¹H de la méthionine dans de l'oxyde de deutérium D₂O avec l'identification de chacun des signaux obtenus.
La figure 4 montre des graphes représentant l'intensité des signaux RMN du proton de la méthionine (signaux 1, 2 et 4 identifiés sur la figure 3) en fonction des équivalents en masse d'unités de répétition du polymère mélangées à la méthionine, en a/ pour le polymère linéaire PL1 conforme à l'invention, en b/ pour le polymère linéaire PL2 conforme à l'invention et en c/ pour le polymère réticulé PR1 conforme à l'invention.
La figure 5 montre un graphe représentant l'intensité des signaux RMN du proton de la méthionine (signaux 1, 2 et 4 identifiés sur la figure 3) en fonction des équivalents en masse d'unités de répétition du polymère mélangées à la méthionine, pour le polymère réticulé PComp1 non conforme à l'invention.
La figure 6 montre un graphe représentant l'absorbance mesurée en fonction de la concentration en dérivé de méthionine fluorescent en mélange avec un polymère réticulé conforme à l'invention PR1.
La figure 7 représente un graphe montrant la concentration totale en méthanethiol (MeSH) et diméthyldisulfure (DMDS) dans un échantillon de vin effervescent ayant été soumis à irradiation après avoir été mis en contact avec un matériau polymère conforme à l'invention, en fonction de la concentration de matériau polymère dans l'échantillon - la ligne en pointillés représente le seuil de perception d'un goût de lumière par un panel sensoriel sur un échantillon de ce même vin irradié et non mis en contact avec le matériau polymère.

### DESCRIPTION DETAILLEE DE L'INVENTION

Le procédé de séquestration de molécules de méthionine contenues dans un milieu liquide selon l'invention met en œuvre un matériau polymère solide à base d'unités monomères de type organométallique, contenant plus particulièrement un noyau porphyrine, modifié ou non pour porter un ou plusieurs substituants, complexé à un atome de cuivre.

Ce matériau polymère est avantageusement choisi pour ne pas être soluble dans le milieu liquide dans lequel il est souhaité de séquestrer les molécules de méthionine. Les polymères à base de noyau porphyrine préconisés par l'invention sont en particulier avantageusement insolubles dans les solutions aqueuses et dans les solutions hydroalcooliques. Il est ainsi possible de les séparer aisément du milieu liquide, ceci sans qu'ils y laissent de résidus, du moins en quantité significative, même après un long temps de contact avec le liquide, par exemple aussi long que le temps d'élevage d'un vin en barrique.

Mis en contact avec les molécules de méthionine contenues dans le milieu liquide, le matériau polymère selon l'invention présente avantageusement une capacité d'adsorption de ces molécules particulièrement élevée, résultant d'une part de la liaison de coordination forte se formant entre les atomes de cuivre et les atomes de soufre des molécules de méthionine, et d'autre part de la structure même des noyaux porphyrine. De manière surprenante, cette capacité d'adsorption est notamment bien plus élevée que celle d'autres polymères de structure proche également sous forme de complexes avec le cuivre.

Outre sa forte capacité de liaison à l'atome de soufre de la méthionine, le cuivre offre les avantages d'une grande abondance, d'un prix raisonnable, et d'une faible toxicité pour les organismes vivants. A titre d'exemple, le seuil maximal de cuivre autorisé dans les vins est élevé, égal à 1 mg/l. Ainsi, lorsque le milieu liquide est une boisson, la mise en contact de cette dernière avec le matériau conforme à l'invention à base de cuivre n'y génère pas, après sa séparation du matériau polymère, de toxicité susceptible d'empêcher sa consommation ultérieure.

Le matériau polymère mis en œuvre selon l'invention présente en outre avantageusement une sélectivité élevée vis-à-vis de la méthionine. En particulier, lorsque le milieu liquide est une boisson, ce matériau n'y adsorbe aucun composé contribuant aux propriétés de cette dernière, et notamment à son profil sensoriel.

Préférentiellement, le matériau polymère solide mis en œuvre est poreux, de préférence avec un fort degré de porosité, correspondant à un ratio surface / volume élevé. Une telle caractéristique augmente plus encore la capacité de piégeage des molécules de méthionine du matériau polymère.

La mise en contact du milieu liquide avec le matériau polymère solide peut être réalisée selon toute méthode classique en elle-même pour la mise en contact d'un solide avec un liquide.

Dans des modes de mise en œuvre particuliers de l'invention, la mise en contact du milieu liquide et du matériau polymère solide est réalisée par incorporation du matériau dans le milieu liquide, et maintien du matériau dans le milieu, de préférence sous agitation, durant un temps de contact suffisant pour permettre l'établissement de liaisons entre le matériau polymère et les molécules de méthionine contenues dans le milieu liquide. Ce temps de contact, qui peut varier en fonction de l'application particulière visée, peut notamment être d'au moins 30 minutes.

Le procédé selon l'invention peut en outre répondre à l'une ou plusieurs des caractéristiques décrites ci-après, mises en œuvre isolément ou en chacune de leurs combinaisons techniquement opérantes.

En particulier, les caractéristiques exactes du matériau polymère solide sont choisies en fonction de l'application visée pour le milieu liquide après que les molécules de méthionine en aient été extraites au moyen du procédé selon l'invention. Lorsque le procédé selon l'invention est destiné au traitement des liquides alimentaires, ces caractéristiques sont ainsi choisies pour répondre aux exigences réglementaires s'appliquant aux composés en contact avec les produits alimentaires.

Le matériau polymère mis en œuvre selon l'invention comporte de préférence au moins trois unités monomères de porphyrine contenant du cuivre. Le nombre exact de telles unités monomères est de préférence choisi pour assurer que le matériau polymère soit insoluble dans le milieu liquide. Il est du ressort de l'homme du métier de déterminer ce nombre minimal d'unités monomères en fonction du milieu liquide particulier concerné.

Dans des modes de mise en œuvre particuliers de l'invention, le polymère contient au moins un groupement hydrophile, qui augmente avantageusement sa compatibilité avec les milieux aqueux.

Ce groupement hydrophile est de préférence un groupement ionique, et préférentiellement anionique. Une telle caractéristique favorise avantageusement le piégeage des molécules de méthionine à la surface du matériau polymère, par formation d'une liaison ionique entre les groupements anioniques portés par le matériau polymère et les groupements amines primaires des molécules de méthionine.

Dans des modes de mise en œuvre préférés de l'invention, le polymère contient au moins un groupement choisi parmi le groupement ion sulfonate ou un de ses sels, le groupement ion carboxylate ou un de ses sels, le groupement ion ammonium ou un de ses sels, ou un groupement acide boronique, le groupement ion sulfonate et ses sels étant particulièrement préférés dans le cadre de l'invention.

Dans des modes de mise en œuvre particuliers de l'invention, au moins un tel groupement hydrophile est de préférence porté par au moins une desdites unités monomères à base de porphyrine contenant du cuivre, notamment au niveau du motif porphyrine, de préférence par plusieurs de ces unités monomères, et préférentiellement chacune d'entre elles.

Dans des modes de mise en œuvre particuliers de l'invention, le noyau de porphyrine d'au moins une desdites unités monomères, de préférence de plusieurs de ces unités monomères, et préférentiellement de toutes, est modifié pour porter au moins un groupement ionique, de préférence anionique, ou un de ses sels, préférentiellement au moins deux groupements ioniques, de préférence anioniques, ou un de leurs sels, les groupements ioniques portés par un même noyau de porphyrine pouvant alors être identiques ou différents.

Autrement, au moins un tel groupement hydrophile peut être porté par un agent de réticulation mis en œuvre pour la préparation du matériau polymère et s'intégrant dans la structure de ce dernier.

Lorsque le groupement hydrophile est un sel d'un groupement ionique, ce sel est préférentiellement choisi pour être compatible avec une utilisation au contact des aliments. Lorsque le groupement ionique est un groupement anionique, il s'agit de préférence d'un sel de métal alcalin, notamment d'un sel de sodium.

Préférentiellement, au moins un groupement anionique porté par le matériau polymère, par exemple par un noyau porphyrine, de préférence chacun des groupements anioniques portés par le matériau polymère, par exemple par les noyaux porphyrine, est un groupement ion sulfonate, le cas échéant sous forme de sel, en particulier de sel de métal alcalin et par exemple de sel de sodium. Un tel groupement ion sulfonate est particulièrement apte à former une liaison ionique avec les groupements amines primaires des molécules de méthionine à pH acide. Il convient en outre à une application au contact alimentaire.

Dans les modes de mise en œuvre de l'invention dans lesquels un ou plusieurs groupements ioniques sont portés par les noyaux porphyrine du polymère, ces groupements ioniques peuvent leur être liés directement, ou par l'intermédiaire d'un bras espaceur. Un tel bras espaceur comporte alors préférentiellement un noyau phényle, sur lequel sont alors de préférence directement fixés le ou les groupements ioniques, notamment le ou les groupements sulfonates.

Dans des modes de mise en œuvre particuliers de l'invention, les noyaux porphyrine du polymère sont modifiés pour porter chacun un ou plusieurs groupements phénylsulfonates. Un groupement sulfonate est alors de préférence présent sur le noyau phényle en position méta et/ou en position para, par rapport au noyau porphyrine.

Préférentiellement, les noyaux porphyrine du matériau polymère ne portent pas groupement de type polyéthylène glycol en tant que seul substituant. De manière plus générale, ils peuvent ne porter aucun groupement de type polyéthylène glycol.

Dans des modes de mise en œuvre particuliers de l'invention, dans le polymère, au moins une des unités monomères, de préférence une pluralité de ces unités monomères, et notamment l'ensemble de ces unités monomères, répond à la formule générale (I) : 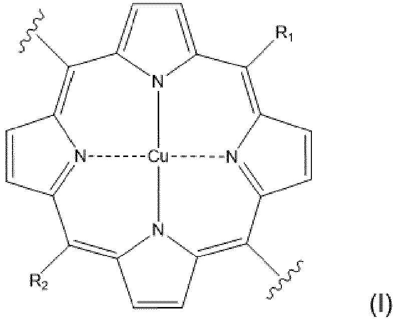 dans laquelle R₁ et R₂, identiques ou différents, représentent chacun un atome d'hydrogène ou un groupement de formule (II) : 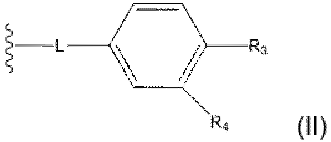 dans laquelle :
R₃ et R₄, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique de préférence un ion sulfonate ou un sel de métal alcalin, par exemple un sel de calcium, d'ion sulfonate,
et L représente une liaison covalente ou un bras espaceur.

Préférentiellement, dans la formule (II), L représente un bras espaceur de formule (III) : 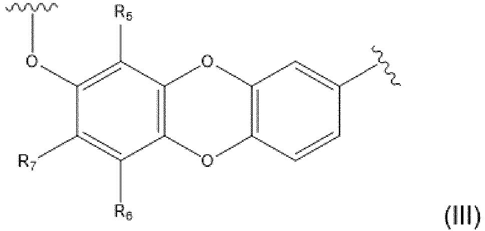 dans laquelle :
R₅ et R₆ représentent chacun un groupement carbonitrile -CN,
et R₇ représente un atome d'halogène, notamment un atome de fluor.

Un tel bras espaceur présente notamment l'avantage d'être rigide et de conduire spontanément à la formation d'une structure poreuse.

Dans la formule (III), l'atome d'oxygène lié au motif 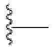 est de préférence relié au noyau phényle du groupement de formule (II), et le noyau phényle lié au motif 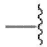 est de préférence lié au noyau porphyrine.

Un tel polymère présente un capacité d'adsorption spécifique de méthionine particulièrement importante. Cette capacité d'adsorption est d'autant plus importante lorsque, dans la formule (II), au moins R₃ ou R₄ représente un groupement ion sulfonate, le cas échéant sous forme de sel.

La liaison des unités monomères au sein du polymère peut être réalisée au moyen de tout agent de réticulation classique en lui-même.

Un tel agent de réticulation peut par exemple être du type à deux groupements carboxylate.

Dans des modes de mise en œuvre particuliers de l'invention, l'agent de réticulation est tel que les unités monomères au sein du polymère sont reliées par un groupe de liaison de formule (IV) : 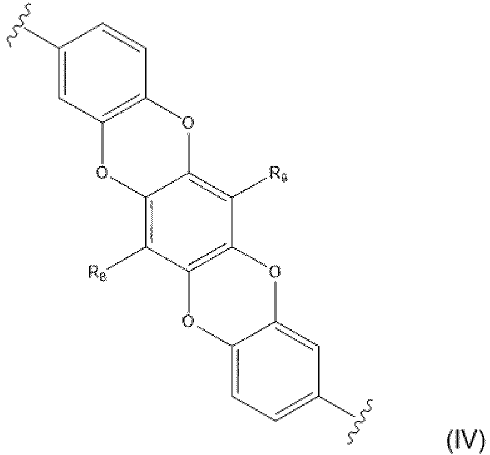 dans laquelle R₈ et R₉ représentent chacun un groupement carbonitrile -CN.

Le polymère mis en œuvre selon l'invention peut être du type linéaire. Il offre alors l'avantage d'une structure parfaitement maitrisée.

En particulier, le polymère mis en œuvre selon l'invention peut répondre à la formule (V) : 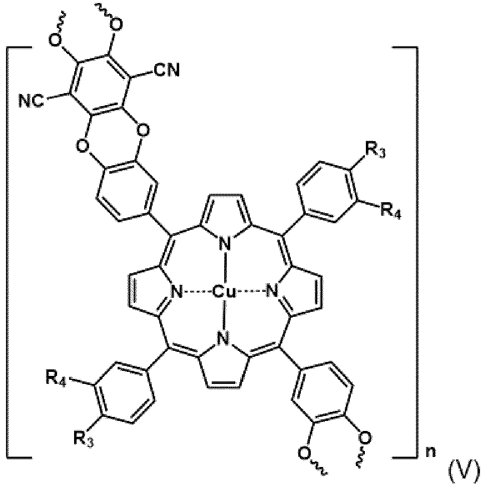 dans laquelle :
R₃ et R₄, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique, l'ion sulfonate et ses sels, de préférence un sel de métal alcalin, par exemple le sel de sodium, étant particulièrement préférés dans le cadre de l'invention,
et n est un nombre entier supérieur à 2, et de préférence inférieur à 1000.

Préférentiellement, dans la formule (V), R₃ représente un atome d'hydrogène et R₄ représente un ion sulfonate ou un de ses sels, de préférence un sel de métal alcalin, par exemple un sel de sodium. Le polymère mis en œuvre selon l'invention peut alors répondre à la formule (Va) : 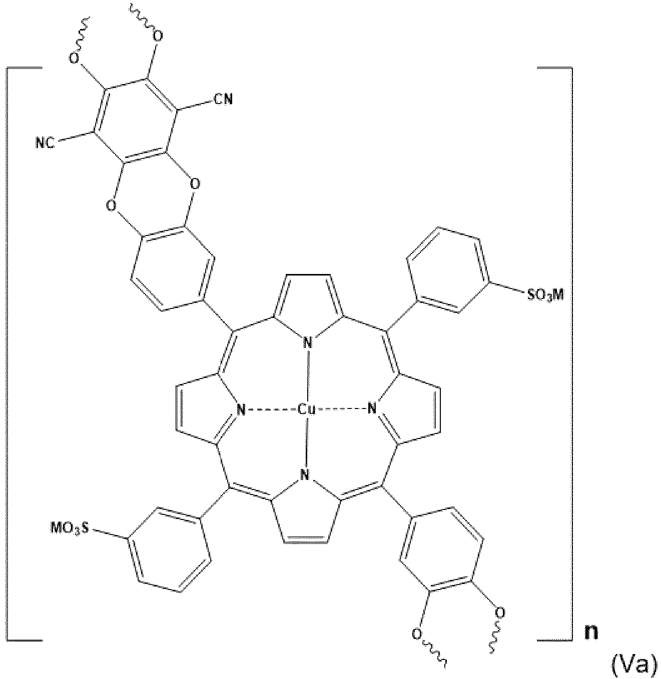 dans laquelle :
M représente un métal alcalin, en particulier le sodium, et
n est un nombre entier supérieur à 2, et de préférence inférieur à 1000.

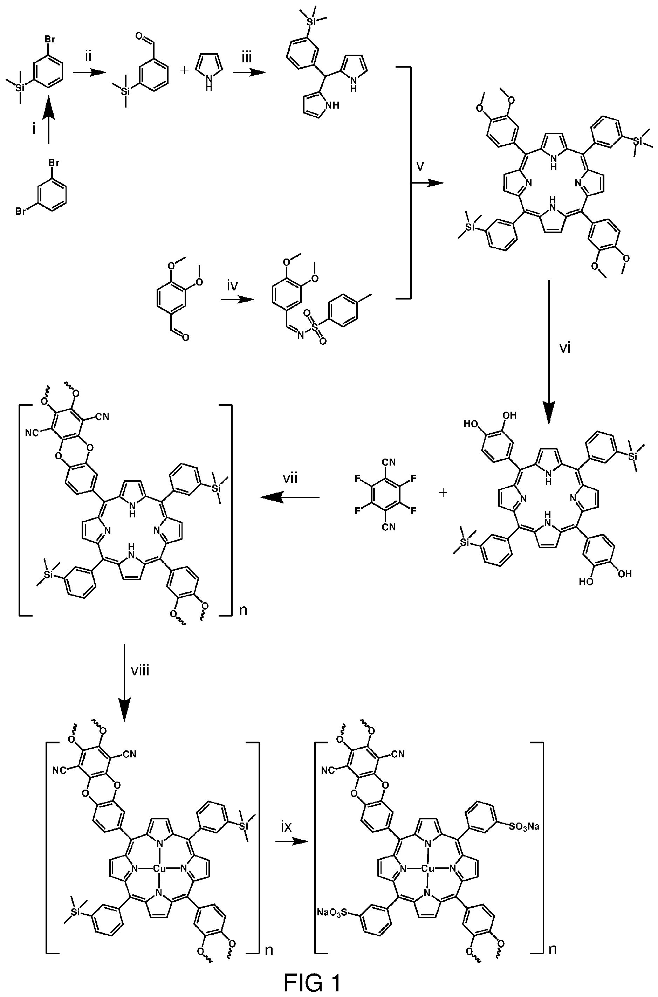

Un tel polymère linéaire peut être préparé selon tout schéma réactionnel dont l'établissement est du ressort de l'homme du métier, par exemple selon le schéma réactionnel montré sur la figure 1, pour le cas dans lequel M représente un atome de sodium. Chacune des étapes i à ix de ce schéma réactionnel peut être mise en œuvre de toute manière classique en elle-même pour l'homme du métier. Un exemple de conditions opératoires est donné à titre d'exemple ci-après dans la présente description.

Dans des modes de mise en œuvre particulièrement préférés de l'invention, le polymère est un polymère réticulé. Un tel polymère, de par notamment sa porosité intrinsèque, présente avantageusement une capacité d'adsorption de la méthionine supérieure à celle des polymères linéaires.

Un tel polymère conforme à l'invention est susceptible d'être obtenu par un procédé de synthèse comprenant des étapes de :
a/ préparation d'un dérivé de porphyrine de formule (VI) : 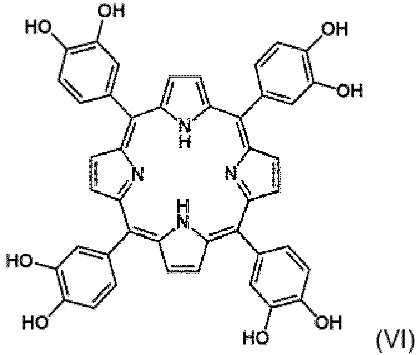
b/ polycondensation du dérivé de porphyrine de formule (VI) avec un agent de réticulation, et
c/ complexation des noyaux porphyrine du polymère obtenu à l'étape b/ avec du cuivre.

Un polymère particulièrement avantageux dans le cadre de l'invention est susceptible d'être obtenu par un procédé de synthèse comprenant des étapes de :
a/ préparation d'un dérivé de porphyrine de formule (VI) : 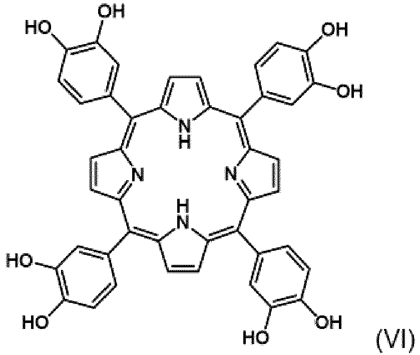
b/ polycondensation du dérivé de porphyrine de formule (VI) avec un composé de formule (VII) : 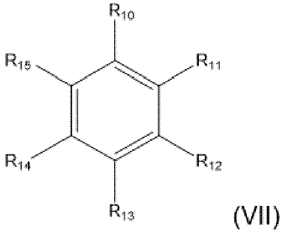 dans laquelle :
   R₁₀ et R₁₃ représentent chacun un groupement carbonitrile -CN,
   R₁₁, R₁₂, R₁₄ et R₁₅, identiques ou différents, représentent chacun un atome d'halogène, notamment un atome de fluor,
   en présence d'un composé de formule (VIII) : 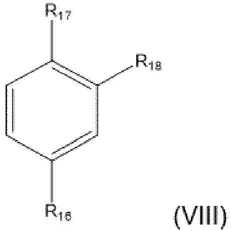 dans laquelle :
      R₁₆ représente un groupement hydroxyle, amine primaire ou secondaire ou thiol, R₁₇ et R₁₈, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique, l'ion sulfonate et ses sels, de préférence un sel de métal alcalin et par exemple le sel de sodium, étant particulièrement préférés selon l'invention,
      de sorte à former un polymère réticulé à branchements aléatoires, et
c/ complexation des noyaux porphyrine du polymère obtenu à l'étape b/ avec du cuivre.

Un tel procédé présente l'avantage d'un nombre d'étapes réduit, et d'une grande facilité et rapidité de mise en œuvre. Il permet en outre avantageusement de former des polymères de haut taux de porosité intrinsèque, le taux de réticulation étant en particulier facilement maitrisable par contrôle des proportions respectives des composés de formules (VI), (VII) et (VIII) lors de l'étape b/ de polycondensation du procédé.

Chacune des étapes du procédé de synthèse du polymère peut être mise en œuvre selon des conditions opératoires classiques en elles-mêmes pour l'homme du métier.

L'étape a/ de préparation du dérivé de porphyrine de formule (VI) peut par exemple être réalisée par une première étape a1/ de condensation de 3,4-diméthoxybenzaldéhyde avec du pyrrole, par exemple dans de l'acide propanoïque à reflux, pour former un premier dérivé de porphyrine de formule (VI'):

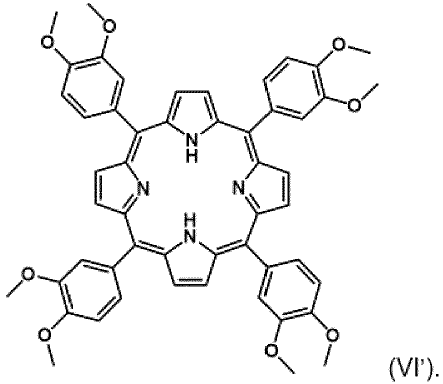

Ce composé de formule (VI') peut ensuite, après purification éventuelle, être soumis à une étape a2/ de déméthylation, par exemple par traitement avec du tribromure de brome, ou un mélange d'acide bromhydrique et d'acide acétique à reflux, afin d'obtenir le dérivé de porphyrine de formule (VI).

L'étape b/ de polycondensation du dérivé de porphyrine de formule (VI) avec le composé de formule (VII), en présence du composé de formule (VIII), menant à la formation d'un polymère réticulé aléatoire, à groupements R₁₇, R₁₈ terminaux, peut par exemple être réalisée par mise en présence des différents réactifs et d'un excès de carbonate de sodium. A titre d'exemple, le rapport molaire des différents composés de formules respectives (VI)/(VII)/(VIII) peut être égal à 1/3/2.

L'étape c/ du procédé de synthèse, de métallation du polymère obtenu à l'issue de l'étape b/, peut quant à elle par exemple être réalisée par mise en contact de ce polymère avec un excès de diacétate de cuivre Cu(OAc)₂ hydraté.

Le polymère réticulé obtenu à l'issue de ce procédé de synthèse est de type aléatoire. Sa structure exacte n'est pas déterminable, il est cependant possible de proposer la formule (IX) suivante pour la structure d'une unité de répétition moyenne de ce polymère : 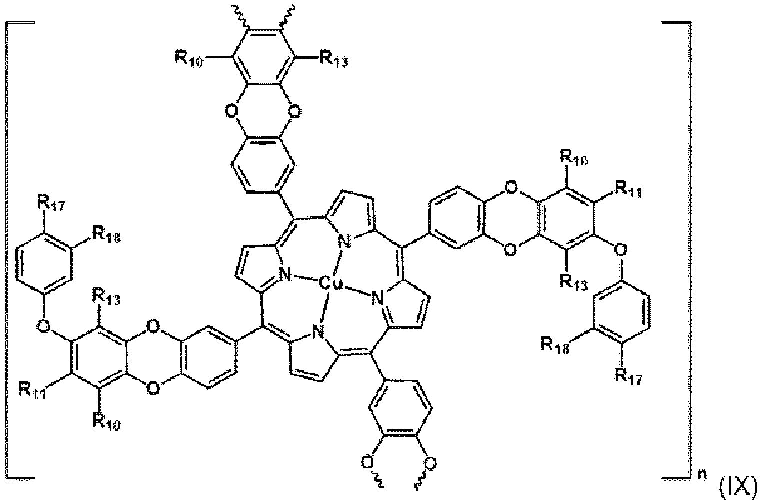 dans laquelle :
R₁₀, R₁₁, R₁₃, R₁₇, R₁₈ sont tels que défini ci-avant, et
n est un nombre entier supérieur à 2, et de préférence inférieur à 1000.

Dans des modes de mise en œuvre particuliers de l'invention :
- dans la formule (VII), R₁₀ et R₁₃ représentent chacun un groupement carbonitrile -CN et R₁₁, R₁₂, R₁₄ et R₁₅ représentent chacun un atome de fluor, le composé de formule (VII) étant alors le 2,3,5,6-tétrafluorotéréphtalonitrile de formule (VIIa) : 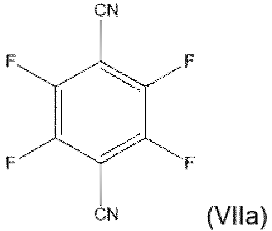
- et/ou, dans la formule (VIII), R₁₆ représente un groupement hydroxyle, R₁₇ représente un sel de sulfonate, de préférence un sel de métal alcalin, par exemple un sel de sodium, et R₁₈ représente un atome d'hydrogène, le composé de formule (VIII) étant alors du phénolsulfonate, de préférence de métal alcalin, notamment de sodium, de formule (VIIIa) : 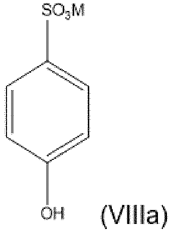
dans laquelle M représente un atome de métal alcalin, par exemple un atome de sodium.

On peut alors proposer, pour le polymère réticulé aléatoire ainsi obtenu, la formule (IXa) suivante pour la structure d'une unité de répétition moyenne de ce polymère : 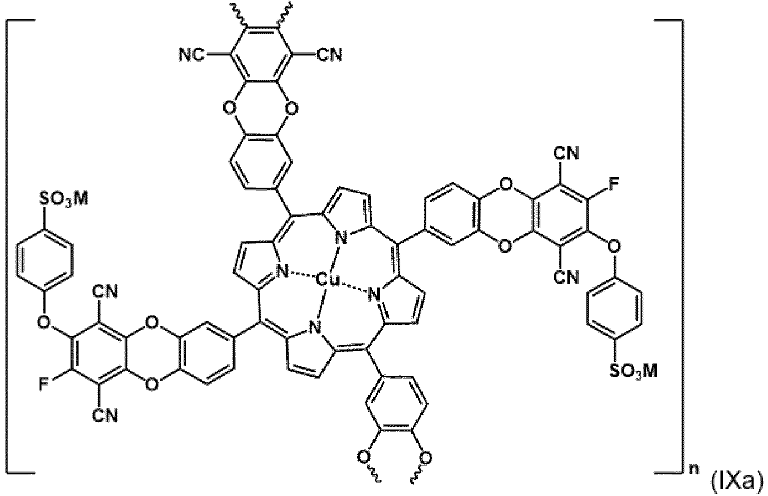 dans laquelle :
M représente un métal alcalin, par exemple le sodium, et
n est un nombre entier supérieur à 2, et de préférence inférieur à 1000.

Un tel matériau polymère présente une capacité de séquestration de la méthionine particulièrement forte et sélective. En particulier, sa constante d'association avec la méthionine est d'environ 3.10⁶ M⁻¹. Sa capacité d'adsorption de la méthionine est quant à elle estimée à environ 250 mg/g de matériau polymère. On ne préjugera pas ici des mécanismes sous-tendant une telle performance. On peut cependant supposer qu'y participent, en combinaison, le fort taux de porosité du matériau, la liaison se formant entre le cuivre (II) contenu dans le polymère et l'atome de soufre de la méthionine, et les interactions électrostatiques se produisant entre les groupements ions sulfonate portés par le polymère et les groupements amine primaire de la méthionine.

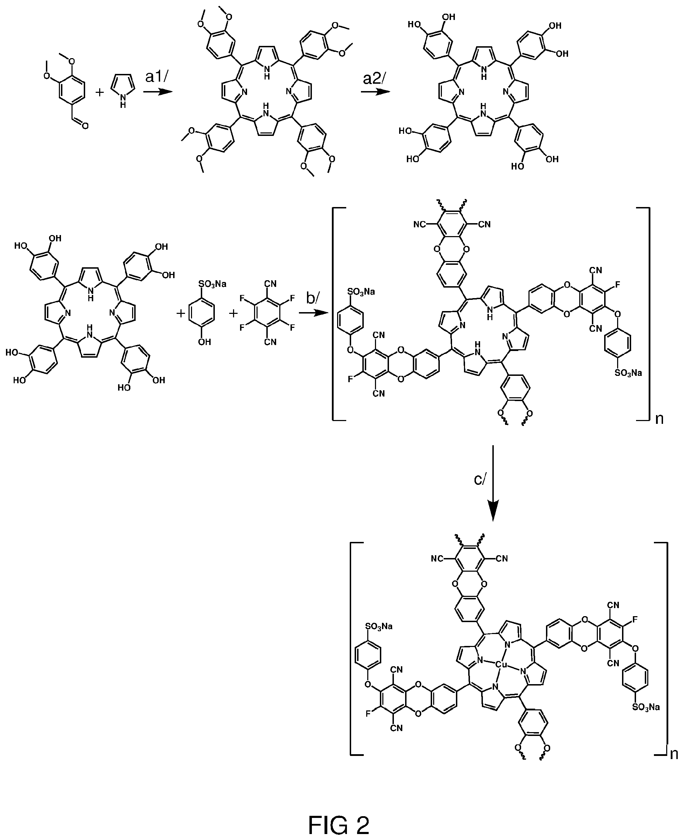

Un exemple de schéma réactionnel permettant d'obtenir un tel matériau polymère réticulé est montré sur la figure 2, pour le cas dans lequel le sel de sulfonate est un sel de sodium.

Dans des modes de mise en œuvre particuliers de l'invention, le matériau polymère est introduit dans le milieu liquide contenant de la méthionine, ou susceptible de contenir de la méthionine, à une dose qui dépend de la quantité de méthionine qu'il est visé de séquestrer, et qui pourra par exemple être comprise entre 0,01 et 50 g/l, notamment comprise entre 0,1 et 10 g/l, et notamment encore comprise entre 0,2 et 2 g/l. A titre d'exemple, lorsque le milieu liquide est un vin effervescent, notamment en cours d'élaboration, en phase de fermentation alcoolique, la dose de matériau polymère introduite dans le milieu liquide peut être d'environ 0,2 g/l, ou plus.

La mise en contact du milieu liquide et du matériau polymère est de préférence réalisée sous agitation.

Le procédé selon l'invention comprend de préférence, après un temps de contact suffisant pour permettre le piégeage des molécules de méthionine par le matériau polymère, une étape de séparation du milieu liquide et du matériau polymère séquestrant la méthionine.

Cette étape de séparation peut être réalisée par toute méthode de séparation solide-liquide connue de l'homme du métier, par exemple par filtration, décantation, dégorgeage lors de la prise en mousse des vins effervescents, etc. Après séparation du milieu liquide, le matériau polymère peut avantageusement être régénéré pour mettre sa réutilisation ultérieure. Ainsi, à cet effet, le procédé selon l'invention peut comprendre une étape finale de séparation des molécules de méthionine et du matériau polymère.

Une telle étape peut par exemple consister en un traitement du matériau polymère par une solution de lavage alcaline, par exemple à pH 10, le cas échéant assisté par ultrasonication. Après séparation du matériau polymère solide de la solution de lavage, et rinçages à l'eau, on obtient un matériau polymère régénéré, présentant à nouveau une forte capacité d'adsorption de méthionine.

Le procédé de séquestration de molécules de méthionine contenues dans un milieu liquide trouve application dans de nombreux domaines.

Une application pour laquelle il s'avère particulièrement adapté est celle du traitement de liquides alimentaires, en particulier destinés à la consommation humaine, en vue d'éviter l'apparition du goût de lumière dans ces derniers consécutivement à leur exposition à la lumière.

Ainsi, un aspect de l'invention concerne un procédé de traitement d'un produit liquide alimentaire, notamment d'une boisson destinée à la consommation humaine, afin d'y diminuer, voire d'y supprimer totalement, le risque d'apparition de goût de lumière. Ce procédé comprend la mise en œuvre, sur ce produit liquide alimentaire, d'un procédé de séquestration de molécules de méthionine contenues dans un milieu liquide selon l'invention, la mise en contact du produit liquide alimentaire avec le matériau polymère étant réalisée pendant au moins 30 minutes, de préférence au moins 2 heures, et étant suivie par une étape de séparation du matériau polymère, séquestrant la méthionine, et du produit liquide alimentaire.

Le produit liquide alimentaire est de préférence choisi parmi les vins, notamment les vins blancs et les vins rosés, ainsi que les effervescents, les produits laitiers tels que le lait et ses dérivés liquides, les produits brassicoles, tels que les bières et les cidres, et tout autre liquide alimentaire contenant de la méthionine et susceptible de contenir de la riboflavine, dans lequel pourrait apparaitre un goût de lumière.

Comme exposé ci-avant, il a été découvert par les présents inventeurs que l'élimination, au moyen du matériau polymère solide conforme à l'invention, des molécules de méthionine présentes dans de tels produits liquides, permet facilement et efficacement d'y éviter la formation de produits soufrés volatiles responsables de l'apparition du goût de lumière, sans pour autant en modifier le profil organoleptique de manière détectable.

Dans le contexte d'une telle application du traitement des boissons pour y prévenir l'apparition du goût de lumière, le temps de contact entre le produit liquide alimentaire et le matériau polymère peut notamment être compris entre 1 jour et plusieurs mois. Il peut par exemple être d'environ 18 mois pour les vins effervescents.

De manière tout à fait avantageuse, dans le cas des vins élevés sur lie et des vins rosés, le matériau polymère peut être introduit directement dans la barrique d'élevage, et maintenu dans cette dernière tout au long de la phase de fermentation alcoolique. Dans le cas des vins effervescents, la mise en contact avec le matériau polymère peut être effectuée lors de la prise de mousse, également en barrique, et maintenue durant toute cette étape. Il a notamment été constaté par les présents inventeurs que le matériau polymère selon l'invention, et notamment les matériaux polymères particuliers tels que décrits ci-avant, peuvent être laissés en contact avec les liquides alimentaires, et notamment les solutions alcooliques, durant des durées aussi longues que plusieurs mois, par exemple de 18 à 24 mois, sans pour autant se dégrader et relarguer dans le produit de résidus indésirables qui ne puissent en être facilement séparés.

Le procédé de séquestration de molécules de méthionine contenues dans un milieu liquide selon l'invention peut également être mis en œuvre sur tout milieu liquide contenant de la méthionine, en vue d'en éliminer cette dernière, par exemple en vue d'une consommation par des individus qui y sont allergiques.

Le procédé de séquestration de molécules de méthionine contenues dans un milieu liquide peut également trouver application dans tout domaine dans lequel il peut être souhaité de détecter la présence de méthionine dans un liquide, voire de doser la méthionine contenue dans un liquide.

Ainsi, un aspect de l'invention concerne un procédé de détection de la présence de méthionine dans un liquide, qui comprend :
- la mise en œuvre, sur un échantillon de ce liquide, d'un procédé de séquestration de molécules de méthionine contenues dans un milieu liquide selon l'invention, la mise en contact de l'échantillon de liquide avec le matériau polymère étant réalisée pendant au moins 30 minutes, notamment comprise entre 30 minutes et 6 heures, et étant suivie par une étape de séparation du matériau polymère, séquestrant le cas échéant de la méthionine, et de l'échantillon de liquide,
- puis l'analyse du matériau polymère ainsi séparé pour la présence de méthionine.

L'étape d'analyse du matériau polymère pour la présence de méthionine peut être réalisée par toute méthode connue de l'homme du métier. Elle peut par exemple être réalisée par séparation de la méthionine et du matériau polymère, notamment comme décrit ci-avant, et analyse de la solution de lavage, isolée du matériau polymère solide, par spectroscopie infrarouge ou spectroscopie par résonnance nucléaire magnétique du proton (RMN ¹H), pour y détecter la présence éventuelle de signaux représentatifs de la méthionine, par exemple respectivement à 2,15 ppm, 2,65 ppm et 3,85 ppm dans des conditions de 300 MHz, D₂O et 298 K.

Optionnellement, l'analyse du matériau polymère pour la présence de méthionine peut comprendre le dosage de la méthionine contenue dans ce matériau polymère.

Un autre aspect de l'invention concerne un matériau polymère adapté à une mise en œuvre dans un procédé selon l'invention de séquestration de molécules de méthionine contenues dans un milieu liquide.

Un tel matériau polymère peut répondre à l'une ou plusieurs des caractéristiques décrites ci-avant en référence à la description du procédé selon l'invention.

Ce matériau polymère est également apte à séquestrer la cystéine.

En particulier, un matériau polymère selon l'invention peut répondre à la formule (V) : 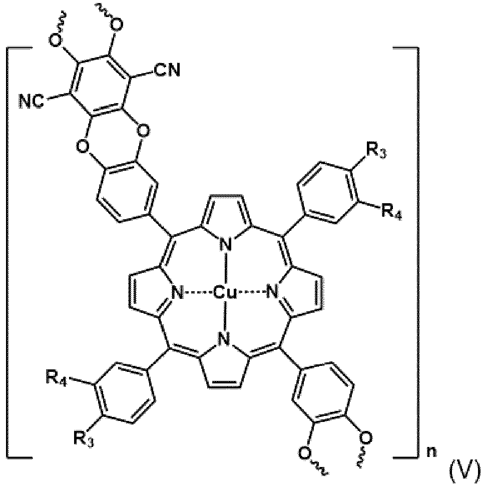 dans laquelle :
R₃ et R₄, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique, l'ion sulfonate et ses sels, en particulier un sel de métal alcalin, par exemple le sel de sodium étant particulièrement préférés selon l'invention,
et n est un nombre entier supérieur à 2, et de préférence inférieur à 1000.

Un autre aspect de l'invention est un matériau polymère solide poreux susceptible d'être obtenu par un procédé de synthèse comprenant des étapes de :
a/ préparation d'un dérivé de porphyrine de formule (VI) : 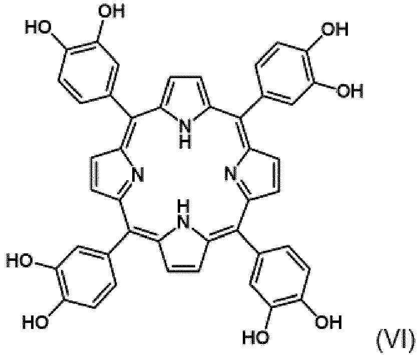
b/ polycondensation dudit dérivé de porphyrine de formule (VI) avec un composé de formule (VII) : 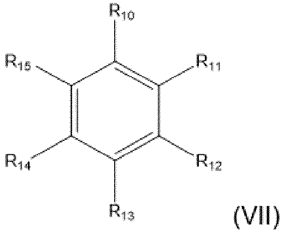 dans laquelle :
   R₁₀ et R₁₃ représentent chacun un groupement carbonitrile -CN,
   R₁₁, R₁₂, R₁₄ et R₁₅, identiques ou différents, représentent chacun un atome d'halogène, notamment un atome de fluor,
   en présence d'un composé de formule (VIII) : 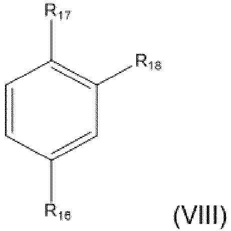 dans laquelle :
      R₁₆ représente un groupement hydroxyle, amine primaire ou secondaire ou thiol, R₁₇ et R₁₈, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique, l'ion sulfonate et ses sels, de préférence un sel de métal alcalin, par exemple le sel de sodium, étant particulièrement préférés dans le cadre de l'invention,
      de sorte à former un polymère réticulé à branchements aléatoires, et
c/ complexation des noyaux porphyrine du polymère obtenu à l'étape b/ avec du cuivre.

Ce procédé de synthèse, et le matériau polymère qu'il permet d'obtenir, peuvent répondre à l'une ou plusieurs des caractéristiques décrites ci-avant en référence au procédé selon l'invention de séquestration de molécules de méthionine contenues dans un milieu liquide.

En particulier :
- dans la formule (VII), R₁₁, R₁₂, R₁₄ et R₁₅ peuvent représenter chacun un atome de fluor,
- et/ou, dans la formule (VIII), R₁₆ peut représenter un groupement hydroxyle, R₁₇ peut représenter un sel de sulfonate, de préférence un sel de métal alcalin, par exemple le sel de sodium, et R₁₈ peut représenter un atome d'hydrogène.

Un matériau polymère solide poreux selon l'invention peut notamment être représenté par la formule (IXa) suivante illustrant la structure d'une unité de répétition moyenne de ce matériau polymère : 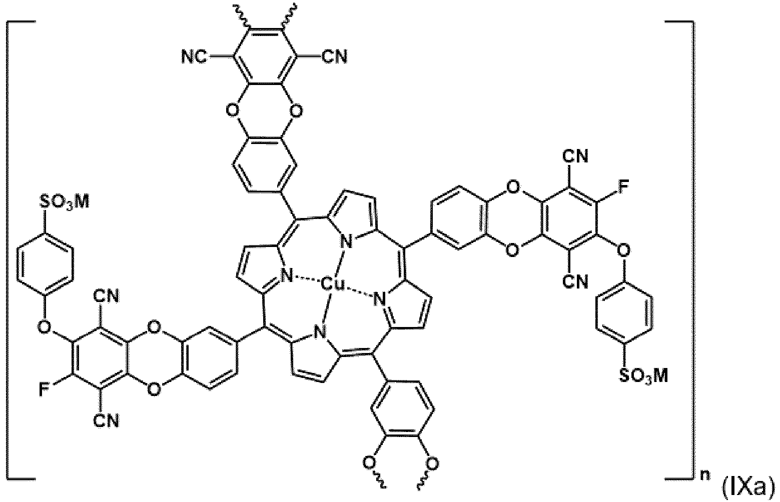 dans laquelle :
M représente un métal alcalin, par exemple le sodium, et
n est un nombre entier supérieur à 2, et de préférence inférieur à 1000.

Un autre aspect de l'invention concerne un procédé de synthèse d'un matériau polymère selon l'invention. Ce procédé répond aux caractéristiques décrites ci-avant en référence à ce matériau polymère.

### EXEMPLES

Les caractéristiques et avantages de l'invention apparaîtront plus clairement à la lumière des exemples ci-après, fournis à simple titre illustratif et nullement limitatifs de l'invention.

### A/ Synthèse de matériaux polymères

Sauf indication contraire, tous les réactifs ont été achetés auprès de sources commerciales et utilisés sans autre purification. Le pyrrole a été distillé juste avant utilisation dans tous les cas. Les spectres RMN ¹H et ¹³C ont été enregistrés sur un spectromètre Bruker UltraShield 300 MHz. Les spectres UV-Visible ont été collectés sur un spectrophotomètre PerkinElmer^{®} Lambda^{®} 750 UV/VIS/NIR.

### A.1/ Polymère linéaire conforme à l'invention - sans groupements sulfonate (PL1)

La synthèse de ce polymère est réalisée selon le schéma réactionnel de la figure 1, à l'exception de la dernière étape qui n'est pas réalisée.

Le 3-triméthylsilylbromobenzène, de formule (X) : 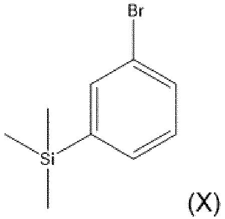 est préparé comme suit. Le 1,3-dibromobenzene (7,12 g, 30,2 mmol) dans le THF (50 mL) et refroidi à -78 °C sous argon. Du n-Butyllithium (1,6 M dans l'hexane, 20,0 mL, 32,6 mmol) est ajouté par seringue et le mélange agité 1 h à -78 °C avant l'ajout de chlorotriméthylsilane (3,61 mL, 33,2 mmol). Après 1,5 h d'agitation à -78 °C, le mélange est ramené à température ambiante et l'agitation continuée pendant la nuit. De l'eau (100 mL) est ajoutée et le mélange est extrait avec le diéthyl éther (3 x 50 mL). Les phases organiques sont lavées à la saumure (50 mL), séchées sur Na₂SO₄, et évaporées sous vide. Le résidu est purifié par chromatographie sur silice (éluant : éther de pétrole). Rendement : 6,27 g (90%).

¹H RMN (300 MHz, 298 K, CDCl₃): δ 7.61 (ddd, J = 2.1, 1.1, 0.5 Hz, 1H, Hₑ), 7.47 (ddd, J = 7.9, 2.1, 1.1 Hz, 1H, Hₐ), 7.42 (dt, J = 7.3, 1.1 Hz, 1H, Hₑ), 7.25 - 7.19 (m, 1H, H_{b}), 0.27 (s, 9H, Hₐ).

Le 3-triméthylsilylbenzaldéhyde, de formule (XI) : 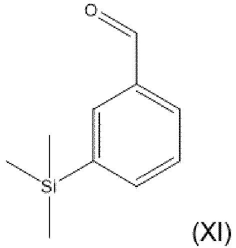 est préparé comme suit. Le 3-bromotriméthylsilylbenzène (X) (6,00 g, 26,2 mmol) est dissous dans le THF (90 mL) et refroidi à -78 °C sous argon. Du n-butyllithium (1,6 M dans l'hexane, 17,7 mL, 28,3 mmol) est ajouté par seringue et le mélange agité 1h à -78 °C avant l'ajout de diméthylformamide (3,25 mL, 41,9 mmol). Après 1 heure à -78 °C, le mélange est porté à température ambiante et HCl (1,0 M, 100 mL) ajouté avec précaution. Le mélange est extrait avec du diéthyl éther (3 x 50 mL). Les phases organiques sont lavées avec une solution saturée en NaHCO₃ (10 mL), saumure (50 mL), séchées sur Na₂SO₄, et évaporées sous vide. Le résidu est purifié par chromatographie sur silice (éluant : éther de pétrole / diéthyl éther). Rendement : 4,22 g (91%). ¹H RMN (300 MHz, 298 K, CDCl₃): δ 10.04 (s, 1H, H_{f}), 8.02 (td, J = 1.6, 0.7 Hz, 1H, Hₑ), 7.85 (ddd, J = 7.6, 1.8, 1.3 Hz, 1H, Hₐ), 7.78 (dt, J = 7.3, 1.3 Hz, 1H, Hₑ), 7.58 - 7.46 (m, 1H, Hₑ), 0.31 (s, 9H, H_{d}).

Le 5-(3-triméthylsilyl)-dipyrrométhane, de formule (XII) : 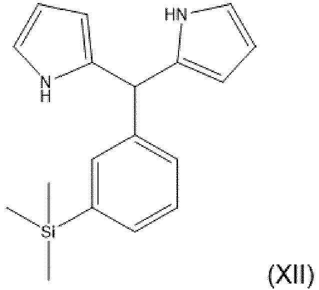 est préparé comme suit. Une solution de 3-trimethylsilylbenzaldehyde (XI) (1,80 g, 10,1 mmol) dans le pyrrole (52,8 mL, 760 mmol) est désaérée avec de l'argon pendant 15 min avant l'ajout d'acide trifluoroacétique (77 µL, 1,01 mmol). Après agitation à l'abri de la lumière pendant 45 min, la triéthylamine (0,5 mL) et le pyrrole sont éliminés par évaporation sous vide. Le produit brut est dissous dans une solution de dichlorométhane/triéthylamine (99:1 v/v) et déposé sur silice pour être purifié par chromatographie (silice, éluant : éther de pétrole/dichlorométhane/triéthylamine 66:33:1). Rendement : 1,18 g (40 %). ¹H RMN (300 MHz, 298 K, CDCl₃): δ 7.94 (s, 2H, Hⱼ), 7.45 - 7.37 (m, 2H, H_{d} et Hₑ), 7.31 (td, *J* = 7.4, 0.7 Hz, 1H, H_{c}), 7.17 (dddd, *J* = 7.6, 1.9, 1.3, 0.5 Hz, 1H, Hₐ), 6.70 (td, *J* = 2.7, 1.6 Hz, 2H, Hᵢ), 6.16 (dt, *J* = 3.4, 2.7 Hz, 2H, Hₕ), 5.92 (d, *J* = 0.8 Hz, 1H, H_{g}), 5.47 (s, 1H, H_{f}), 0.24 (s, 9H, H_{b}).

La 3,4-diméthoxybenzène-4-N-tosylimine, de formule (XIII) : 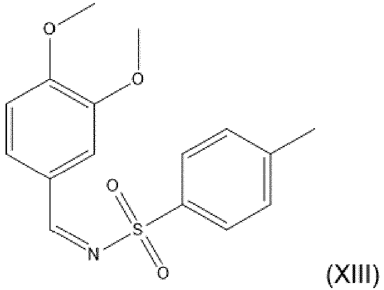 est préparée comme suit. L'acide p-toluènesulfonique monohydrate (0,622 g, 3,61 mmol) est ajouté à une solution de 3,4-diméthoxybenzaldéhyde (6,00 g, 36,1 mmol) et p-toluénesulfonamide (6,18 g, 36,1 mmol) dans le toluène (100 mL) et le mélange porté à reflux dans un montage Dean-Stark pendant 3 j. Après retour à température ambiante, l'acide est neutralisé par ajout de triéthylamine (3 mL) et le solvant évaporé sous vide. Le produit est purifié par chromatographie sur silice (éluant: dichlorométhane/éther de pétrole 2:1) et recristallisé (diéthyl éther/acétate d'éthyle 2:1). Rendement: 7,81 g (68%).

¹H RMN (300 MHz, 298 K, CDCl₃): δ 8.91 (s, 1H, H_{f}), 7.93 - 7.83 (m, 2H, H_{g}), 7.51 (d, J = 1.9 Hz, 1H, Hₑ), 7.43 (dd, J = 8.3, 2.0 Hz, 1H, H_{d}), 7.38 - 7.28 (m, 2H, Hₕ), 6.93 (d, J = 8.3 Hz, 1H, Hₑ), 3.96 (s, 3H, H_{b}), 3.91 (s, 3H, Hₐ), 2.43 (s, 3H, Hᵢ).

La 5,15-di-(3-triméthylsilylphényl)-10,20-di-(3,4-diméthoxyphényl) porphyrine, de formule (XIV) : 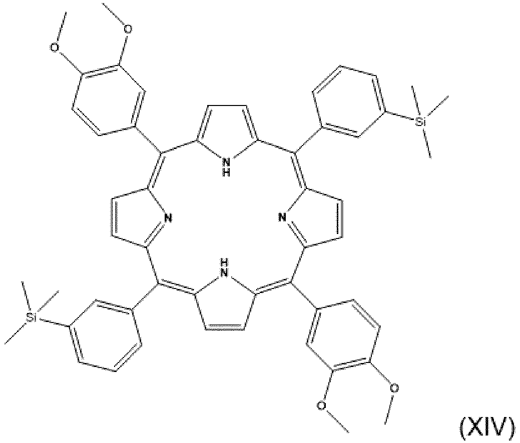 est préparée comme suit. Le 3,4-diméthoxybenzène-4-N-tosylimine (XIII) (1,19 g, 3,74 mmol) et le triflate de cuivre (II) (135,0 mg, 0,374 mmol) sont dissous sous argon dans le dichlorométhane (150 mL). Une solution de 5-(3-triméthylsilylphényl)dipyrrométhane (XII) (1,10 g, 3,74 mmol) dans le dichlorométhane sous argon (150 mL) y est ajouté à l'abri de la lumière. Après agitation à température ambiante (1,5 h), le DDQ (2,3-dichloro-5,6-dicyano-1,4-benzoquinone) (1,70 g, 7,47 mmol) est ajouté el le mélange agité pendant la nuit. De l'eau (50 mL) est ajoutée et le mélange est extrait avec le dichlorométhane (3 x 50 mL). Les phases organiques sont séchées sur Na₂SO₄, et évaporées sous vide. Le résidu est purifié par chromatographie sur silice en collectant la deuxième fraction (éluant : dichlorométhane). Rendement : 380 mg (23%).

¹H RMN (300 MHz, 298 K, CDCl₃): δ 8.88 (dd, J = 19.0, 4.80 Hz, 8H, H_{g} et Hₕ), 8.36 (d, J = 4.2 Hz, 2H, Hₗ), 8.19 (m, 2H, Hᵢ), 7.92 (dt, J = 7.4, 1.3 Hz, 2H, Hₖ), 7.75 (m, 6H, H_{d}, Hₑ, et Hⱼ), 7.26 (m, 2H, Hₑ), 4.18 (s, 6H, H_{b}), 3.99 (s, 6H, Hₐ), 0.41 (s, 18H, Hₘ), -2.73 (s, 2H, H_{f})

ESI-MS (MeOH): *m*/*z* = 879.37285 (calc. pour C₅₄H₅₅N₄O₄Si₂⁺ 879.37564).

La 5,15-di-(3-triméthylsilylphényl)-10,20-di-(3,4-dihydroxyphényl) porphyrine, de formule (XV) : 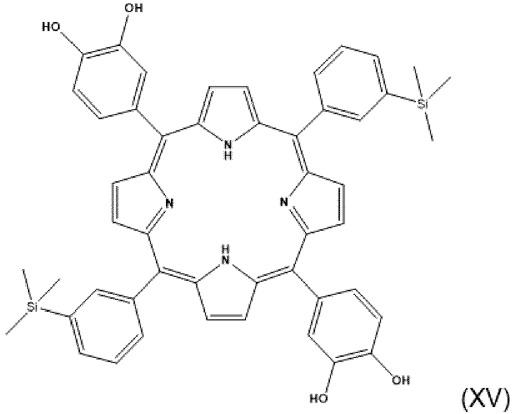 est préparée comme suit. La 5,15-di-(3-triméthylsilylphényl)-10,20-di-(3,4-diméthoxyphényl) porphyrine (XIV) (125 mg, 0,142 mmol) est dissoute dans le dichlorométhane (DCM) (25 mL) sous argon et le mélange est refroidi à -80 °C. Du tribromure de bore BBr₃ (1,0 M dans le DCM, 1,14 mmol) est ajouté par une seringue et le mélange est agité à -80 °C pendant 3 h puis à température ambiante pendant une nuit. Du méthanol (10 mL) est ajouté, suivi d'eau (10 mL) et d'acétate d'éthyle (30 mL). La phase aqueuse est neutralisée par ajout de solution aqueuse saturée de NaHCO₃ et extraite avec l'acétate d'éthyle (3 x 20 mL). Les phases organiques sont combinées et lavées à l'eau (20 mL), saumure (20 mL), puis séchées sur Na₂SO₄. Après évaporation des solvants, le produit est recristallisé dans l'acétone/hexane et séché sous vide. Rendement: 153 mg, 81%.

¹H RMN (300 MHz, Acétone) δ 8.92 (dd, *J* = 46.2, 4.9 Hz, 8H, H_{g} et Hₕ), 8.37 (s, 2H, Hₗ), 8.30 - 8.21 (m, 2H, Hᵢ), 8.02 (dt, *J* = 7.4, 1.2 Hz, 2H, Hₖ), 7.91 - 7.80 (m, 2H, Hⱼ), 7.74 (d, *J* = 2.2 Hz, 2H, Hₑ), 7.57 (dd, *J* = 8.0, 2.1 Hz, 2H, H_{d}), 7.27 (d, *J* = 8.0 Hz, 2H, Hₑ), 0.44 (s, 18H, Hₘ), -2.71 (s, 2H, H_{f})

ESI-MS (MeOH): *m*/*z* = 823.11421 (calc. pour C₅₄H₅₅N₄O₄Si₂⁺ 823.11200).

La polymérisation du dérivé de porphyrine de formule (XV) est réalisée comme suit, pour former le polymère de formule (XVI) : 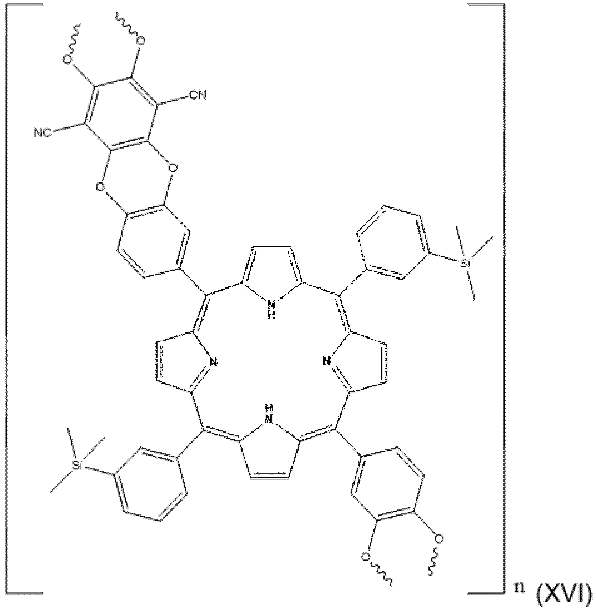 dans laquelle n est un nombre entier compris entre 3 et 999.

La 5,15-di-(3-triméthylsilylphényl)-10,20-di-(3,4-dihydroxyphényl) porphyrine (0,100 g, 0,121 mmol), et du carbonate de potassium (0,201 g, 1,46 mmol, 12 éq.) sont combinés dans un ballon sous argon. Du DMF anhydre (12 mL) est ajouté et le mélange porté à 80 °C sous agitation. Après quelques minutes, le 2,3,5,6-tetrafluorotéréphthalonitrile (0,024 g, 0,121 mmol, 1 éq.) dissous dans du DMF (10 mL) est ajouté sur 20 min par une seringue. Le mélange est porté à reflux pendant 2 jours, refroidi, et dilué avec de l'eau (100 mL) sous ultrasons pendant 10 min. La suspension est filtrée, lavée à l'eau, acétone, méthanol, THF, dichlorométhane et acétone (2 x 10 mL). Le solide obtenu est séché à 55 °C sous vide. Rendement : 104 mg (90%).

Le polymère linéaire PL1 conforme à l'invention, répondant à la formule (XVII) : 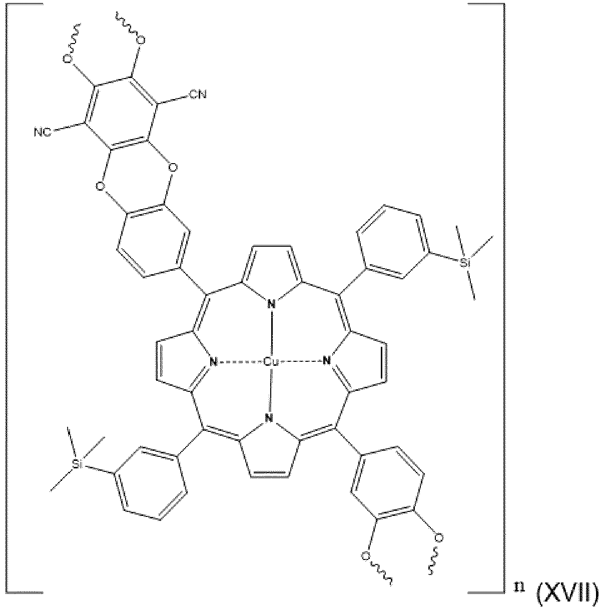 dans laquelle n est un nombre entier compris entre 3 et 999,
est obtenu par métallation du polymère de formule (XVI), par mise en suspension de ce dernier (0,060 g) dans de l'eau (7 ml) et ajout de Cu(OAc)₂ (0,064 g, 0,318 mmol). Le mélange a été agité à 50 °C pendant une nuit, refroidi, filtré et rincé avec de l'eau puis de l'acétone (2 x 10 ml chacun), séché à 55 °C sous vide. Rendement : 50 mg (78 %).

### A.2/ Polymère linéaire conforme à l'invention - avec groupements sulfonate (PL2)

Le polymère PL2 conforme à l'invention, de formule (XVIII) : 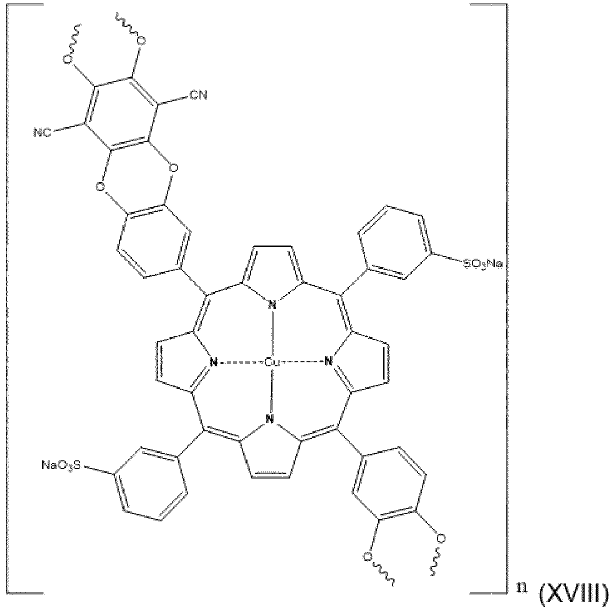 dans laquelle n est un nombre entier compris entre 3 et 999,
est préparé à partir du polymère de formule (XVII), selon la dernière étape du schéma réactionnel représenté sur la figure 1. Ce dernier (0,040 g) est suspendu dans du tétrachlorométhane CCl₄ (4 mL) et du triméthylsilylchlorosulfonate (0,0386 mL, 0,239 mmol) est ajouté par seringue. Le mélange est porté à reflux pendant 18 h, refroidi, et une solution de NaOH (1 M, 5 mL) et de bicarbonate de sodium saturée (5 mL) sont ajoutées. Le mélange est agité vigoureusement pendant 1 h et le solide obtenu est filtré et lavé à l'eau, acétone, dichlorométhane, et méthanol (2 x 10 mL chacun) avant d'être séché à 55 °C sous vide. Rendement : 38 mg (90%).

Analyse élémentaire : attendu C, 56.96; H, 2.02; N, 7.67; S, 5.85; Cu, 5.80; trouvé C, 51.35; H, 2.51; N, 7.35; S, 5.77; Cu, 5.70.

### A.3/ Polymère réticulé non conforme à l'invention - sans cuivre (Pcomp1)

La synthèse de ce polymère est réalisée selon le schéma réactionnel de la figure 2, à l'exception de la dernière étape qui n'est pas réalisée.

La tétra-(5,10,15,20-(3,4-diméthoxyphényl))porphyrine, de formule (XIX) : 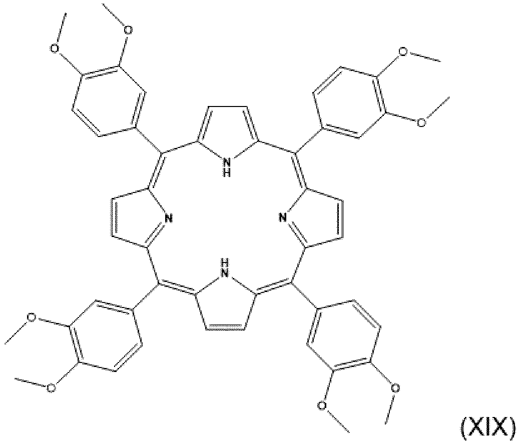
est préparée par ajout goutte à goutte de pyrrole (0,63 mL, 9,03 mmol) à une solution de 3,4-diméthoxybenzaldéhyde (1,50 g, 9,03 mmol) dans l'acide propionique (35 mL) porté à reflux (à environ 170 °C). Le mélange est chauffé à reflux à l'air pendant 30 min et laissé refroidir à température ambiante. Le solvant est évaporé sous vide et le résidu obtenu purifié par chromatographie sur silice (éluant : dichlorométhane) et par re-précipitation dans le méthanol. Rendement : 310 mg (15%).
¹H RMN (300 MHz, 298 K, CDCl₃): δ 8.90 (s, 8H, H_{f}), 7.80 - 7.74 (m, 8H, H_{d} et Hₑ), 7.31 - 7.22 (m, 4H, Hₑ), 4.18 (s, 12H, H_{b}), 3.99 (s, 12H, Hₐ), -2.73 (s, 2H, H_{g})
ESI-MS (MeOH): 855.33620 (calc. pour C₅₂H₄₇N₄O₈⁺ 855.33884).

La tétra-(5,10,15,20-(3,4-dihydroxyphényl))porphyrine, de formule (XX) : 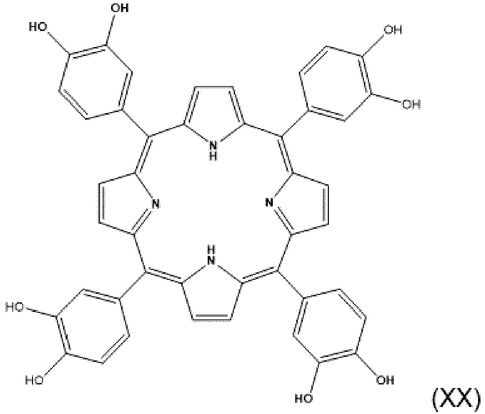
est préparée comme suit. Une solution de tétra-(5,10,15,20-(3,4-diméthoxyphényl))porphyrine (XIX) (300 mg, 0,351 mmol) dans un mélange de bromure d'hydrogène HBr concentré (20 mL) et acide acétique glacial (15 mL) est portée à reflux pendant 3 jours. Le mélange obtenu est ensuite refroidi et dilué avec de l'acétate d'éthyle (100 mL) et eau (50 mL). Après neutralisation avec une solution saturée de NaHCO₃, le mélange est extrait avec l'acétate d'éthyle (4 x 50 mL), et les phases organiques lavées à l'eau, saumure, et séchées sur Na₂SO₄. Après évaporation des phases volatiles, le solide obtenu est purifié par recristallisation (acétone / hexane) pour donner des cristaux violets. Rendement : 226 mg (86%).
¹H RMN (300 MHz, 298 K, de-acétone): δ 8.96 (s, 8H, H_{f}), 7.74 (d, J = 2.1 Hz, 4H, Hₑ), 7.56 (dd, J = 8.0, 2.1 Hz, 4H, H_{d}), 7.26 (d, J = 8.0 Hz, 4H, Hₑ), -2.73 (s, 2H, H_{g})
ESI-MS (MeOH): 743.21308 (calc. pour C₄₄H₃₁N₄O₈⁺ 743.21364)
UV-Vis (MeOH): λₘₐₓ (nm) (ε (x10³ M⁻¹cm⁻¹)) 422 (275), 518 (13.0), 556 (9.83), 594 (4.89), 650 (5.06).

Le polymère réticulé Pcomp1, dont la structure d'une unité monomère moyenne peut être représentée par la formule (XXI) : 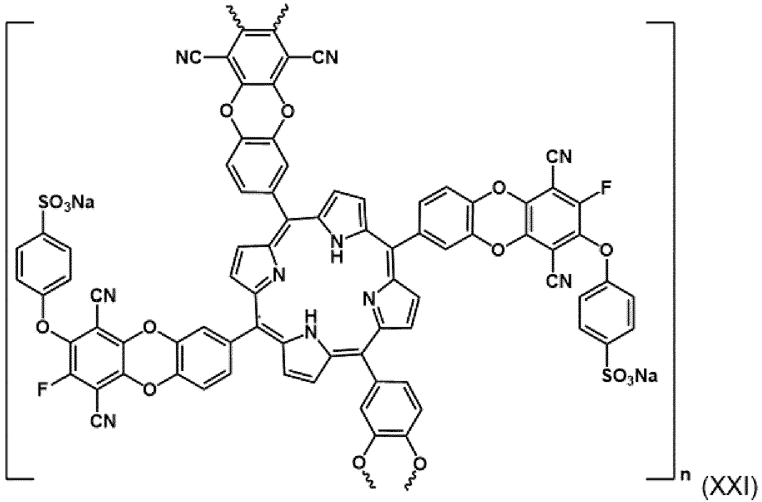 dans laquelle n est un nombre entier compris entre 3 et 999,
est obtenu par polymérisation du composé de formule (XX). Ce dernier (1,00 g, 1,35 mmol), du 4-phénol sulfonate de sodium (0,528 g, 2,69 mmol, 2 éq.) et du carbonate de sodium (4,28 g, 40,4 mmol, 30 éq.) sont combinés dans un ballon sous argon. Du DMF anhydre (50 mL) est ajouté et le mélange porté à 80 °C sous agitation. Après quelques minutes, du 2,3,5,6-tétrafluorotéréphthalonitrile (0,808 g, 4,04 mmol, 3 éq.) dissous dans du DMF (10 mL) est ajouté sur 20 min par une seringue. Le mélange est porté à reflux pendant 2 jours, refroidi, et dilué avec de l'eau (100 mL) sous ultrasons pendant 10 min. La suspension est filtrée, lavée à l'eau, acétone, méthanol, THF, dichlorométhane et acétone (2 x 10 mL). Le solide obtenu est séché à 55 °C sous vide. Rendement : 1,39 g (67%).

### A.4/ Polymère réticulé conforme à l'invention (PR1)

Le polymère réticulé PR1 est obtenu à partir du polymère Pcomp1 selon la dernière étape du schéma réactionnel montré sur la figure 2. On peut illustrer la structure d'une unité monomère moyenne de ce polymère par la formule (XXII) : 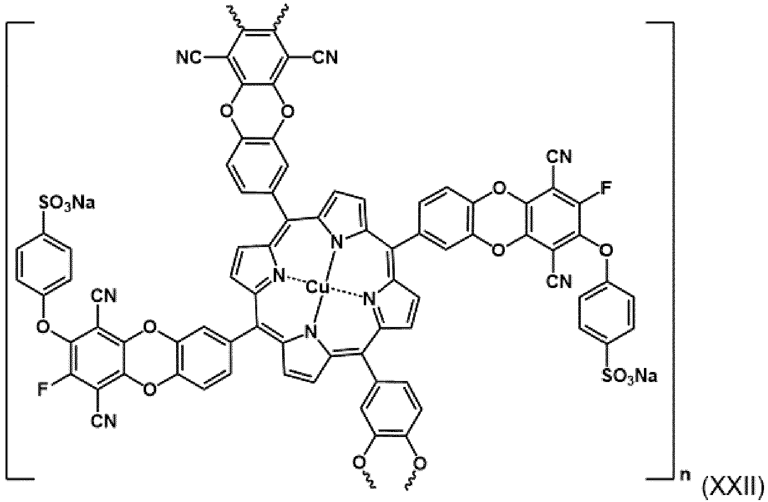 dans laquelle n est un nombre entier compris entre 3 et 999.

A cet effet, le polymère Pcomp1 (1,39 g) est suspendu dans de l'eau (15 mL) et du diacétate de cuivre (II) Cu(OAc)₂ (0,927 g, 4,64 mmol) est ajouté. Le mélange est agité 16 h à 50 °C, filtré, lavé à l'eau et à l'acétone avant d'être séché à 55 °C sous vide. Rendement : 1.40 g (96%).

Analyse élémentaire : attendu C, 60.18; H, 1.77; N, 8.77; S, 4.02; Cu, 3.98 ; trouvé C, 56.63; H, 1.88; N, 10.11; S, 1.03; Cu, 6.0.

### A.5/ Polymère non conforme à l'invention - sans motifs porphyrine (Pcomp2)

Un polymère non conforme à l'invention, sans motif porphyrine, à unités monomères à base de ligand salen contenant du cuivre et d'un groupement éther couronne, connu pour sa capacité à se lier aux groupements amines des acides aminés, est également préparé.

Le composé de formule (XXIII) : 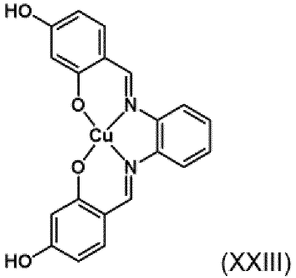 est préparé par ajout goutte à goutte d'une solution d'éthanol (10 mL) contenant du 2,4-dihydroxybenzaldéhyde (0,500 g, 3,62 mmol) à une solution de 1,2-diaminobenzène (0,196 g, 1,81 mmol) dans l'éthanol (5 mL). Le mélange est porté à reflux pendant 2 h et une solution de diacétate de cuivre monohydrate (0,361 g, 1,81 mmol) dans l'éthanol (10 mL) est ajoutée lentement. Le reflux est arrêté après 2 h et le précipité brun est collecté par filtration et lavé à l'éthanol froid (2 x 10 mL), puis acétone (2 x 10 mL) et séché sous vide à 70 °C. Rendement : 670 mg, 90%.

ESI-MS (MeOH): 410.03198 (calc. pour C₂₀H₁₅N₂O₄Cu⁺ 410.03223).

Le composé de formule (XXIV) : 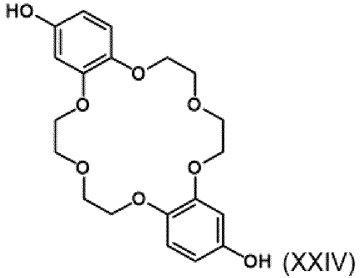 est quant à lui préparé comme suit.

Du di-(méta-acéto-)benzo-éther-18-couronne-6 est tout d'abord préparé par mélange d'acide acétique glacial (0,875 g, 15,3 mmol) et de réactif de Eaton (29,3 mL, soit 3,3 g, 11,9 mmol de pentoxyde de phosphore) et agitation sous argon pendant 15 min. Le dibenzo-éther-18-couronne-6 (2,00 g, 5,10 mmol) y est ajouté en une portion et le mélange est agité à 50 °C pendant une nuit avant d'être versé dans 150 mL d'eau et glace pilée. Le mélange est extrait avec le DCM (4 x 50 mL), et les phases organiques sont lavées à l'eau (2 x 50 mL) et séchées sur Na₂SO₄. Après évaporation des solvants, le résidu est purifié par chromatographie sur alumine (éluant : chloroforme). Rendement : 1,50 g, 65%. ¹H RMN (300 MHz, CDCl₃) δ 7.54 (dd, J = 8.4, 2.0 Hz, 2H, Hₑ), 7.49 (d, J = 2.0 Hz, 2H, H_{d}), 6.84 (d, J = 8.3 Hz, 2H, H_{b}), 4.22 (dd, J = 5.5, 3.2 Hz, 8H, H_{f} et H_{g}), 4.03 (dt, J = 8.6, 4.7 Hz, 8H, Hₑ et Hₕ), 2.54 (s, 6H, Hₐ).

Le composé obtenu (1,00 g, 2,25 mmol) est dissous dans le DCM (15 mL) et de l'acide tosylique hydraté (0,642 g, 3,38 mmol) est ajouté. Le mélange est purgé à l'argon pendant 10 min et de l'acide métachloroperbenzoïque m-CBPA (environ 50% 3,1 g, 9,00 mmol) est ajouté. Le mélange est agité une nuit et filtré sur la Celite^{®}. Le filtrat est lavé avec une solution aqueuse de NaHSO₃ (2 x 30 mL), eau, et saumure avant d'être séchée sur Na₂SO₄. Après évaporation des solvants, le produit obtenu, di-(méta-acétato-)benzo-éther-18-couronne-6, est pur à plus de 90%. Rendement : 550 mg (environ 50%).

¹H RMN (300 MHz, CDCl₃, 298 K) δ 6.84 (d, *J* = 9.3 Hz, 2H, Hₑ), 6.62 (m, 4H, H_{b} et H_{d}), 4.14 (m, 8H, Hₑ et Hₕ), 4.01 (m, 8H, H_{f} et H_{g}), 2.27 (s, 6H, Hₐ).

Le produit obtenu (0,570 g, 1,20 mmol) est dissous dans un mélange de dichlorométhane/méthanol (7:11 v/v, 55 mL) à l'aide d'ultrasons, et la solution désaérée. De l'hydroxyde de sodium (0,269 g, 6,72 mmol) est dissous dans le méthanol (8 mL) et désaéré (argon) avant d'être ajouté et le mélange agité à température ambiante pendant une nuit. HCl concentré est ajouté jusqu'à pH neutre et le précipité blanc est collecté par filtration. Le produit de formule (XXIV) obtenu est pur à plus de 90% et utilisé tel quel. Rendement : 420 mg (environ 90%).

¹H RMN (300 MHz, CDCl₃, 298 K) δ 6.78 (d, *J* = 8.7 Hz, 2H, ), 6.49 (d, *J* = 2.7 Hz, 2H, ), 6.33 (dd, *J* = 8.7, 2.7 Hz, 2H, ), 4.18 - 4.08 (m, 8H, Hₑ et Hₕ), 3.97 (ddt, *J* = 6.7, 4.6, 2.2 Hz, 8H, H_{f} et H_{g}).

Le polymère Pcomp2, de formule (XXV) : 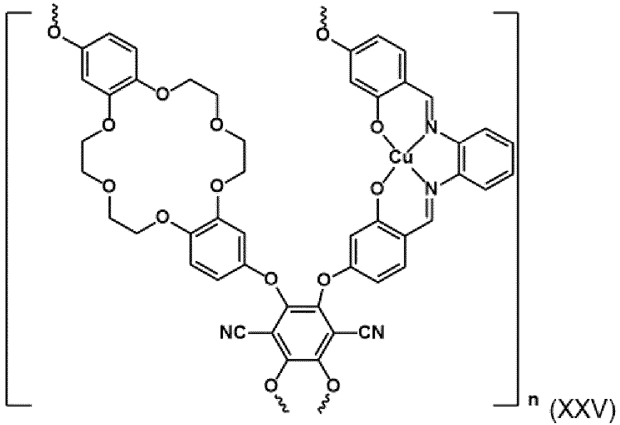 dans laquelle n est un nombre entier compris entre 3 et 999,
est préparé comme suit.

Le composé de formule (XXIII) (40 mg, 0,098 mmol), le composé de formule (XXIV) (42,1 mg, 0,107 mmol) et du carbonate de sodium (82,7 mg, 0,781 mmol) sont combinés dans un ballon sous argon. Le DMF anhydre (3 mL) est ajouté et le mélange porté à 80 °C sous agitation. Après quelques minutes, du 2,3,5,6-tétrafluorotéréphthalonitrile (19,5 mg, 0,098 mmol, 1 éq.) dissous dans le DMF (10 mL) est ajouté sur 20 min par une seringue. Le mélange est porté à reflux pendant 2 jours, refroidi, et dilué avec de l'eau (100 mL) sous ultrasons pendant 10 min. La suspension est filtrée, lavée à l'eau, acétone, méthanol, THF, dichlorométhane et acétone (2 x 10 mL). Le solide obtenu est séché à 55 °C sous vide. Rendement : 72 mg (77%).

### B/ Tests de liaison à la méthionine

### B.1/ Etude de la complexation de la méthionine par RMN ¹H

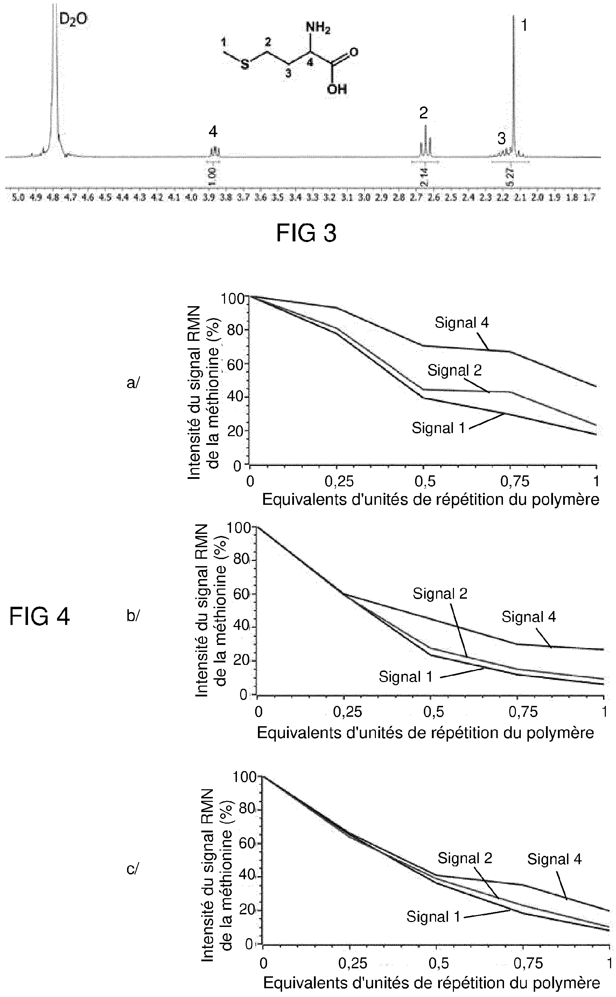

La capacité des différents polymères à absorber la méthionine a été évaluée par spectroscopie par résonance magnétique nucléaire du proton RMN ¹H. Cela a été fait en dissolvant 0,15 mg de méthionine dans 0,5 ml de D₂0 (concentration : 2 mM) et en surveillant la diminution de l'intensité du signal des résonances de cet acide aminé lors d'ajouts successifs de portions du polymère. La masse d'un équivalent des unités répétées de polymère estimées a été calculée et 0,25 éq. de polymère ont été ajoutés à chaque ajout, jusqu'à atteindre 1,0 éq. Cela visait à garantir que la quantité de méthionine absorbée par chaque polymère pouvait être comparée par ion cuivre (un par unité de répétition). Après chaque addition de 0,25 éq. de polymère, les tubes RMN ont été soumis à des ultrasons pendant 9 min, la résine a pu se déposer au fond du tube RMN pendant environ 5 min, puis le spectre RMN a été enregistré. Le système de marquage de signal utilisé est montré sur la figure 3.

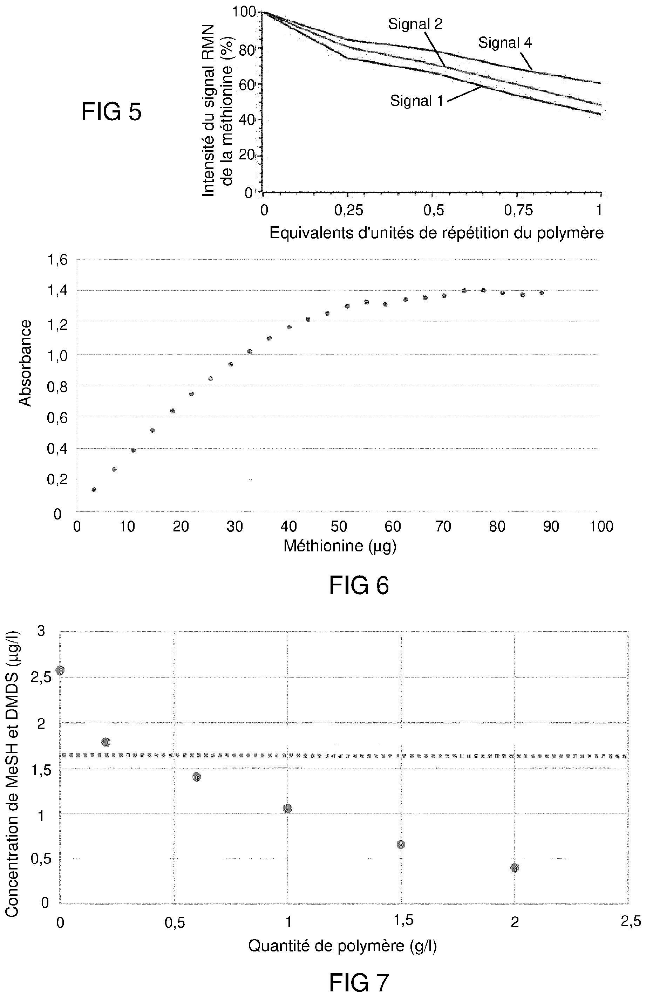

Les résultats obtenus pour les signaux représentatifs de la méthionine 1, 2 et 4, en fonction des équivalents d'unités de répétition du polymère, sont montrés sur la figure 4, en a/ pour le polymère PL1, en b/ pour le polymère PL2 et en c/ pour le polymère PR1, et sur la figure 5 pour le polymère Pcomp1. Concernant le polymère Pcomp2, les intensités du signal de la méthionine ayant diminué de moins de 10 % après l'ajout de 1,0 éq. du polymère, les courbes n'ont pas été tracées.

On observe sur la figure 4, pour chacun des polymères conformes à l'invention testés, un diminution importante des signaux RMN de la méthionine, témoignant d'une importante séquestration de la méthionine dans le polymère. Parmi les polymères linéaires, la séquestration est plus importante pour le polymère PL2, dans lequel les noyaux porphyrine portent des groupements ion sulfonate (86 % en moyenne de diminution des signaux RMN à 1 éq.), que pour le polymère PL1 qui en est dépourvu (71 % en moyenne de diminution des signaux RMN à 1 éq.). Le polymère séquestrant la quantité la plus importante de méthionine est le polymère réticulé PR1 (88 % en moyenne de diminution des signaux RMN à 1 éq.). Ce polymère présente en outre les avantages d'être facile et rapide à synthétiser, en 4 étapes seulement, et avec un bon rendement. En comparaison, comme montré sur la figure 5, le polymère réticulé de structure similaire mais dépourvu de cuivre (PComp1) présente une capacité largement moindre de séquestration de la méthionine (50 % en moyenne de diminution des signaux RMN à 1 éq.), insuffisante pour nombre d'applications. Le polymère PComp2, à base de ligand salen, cuivre et éther 18-couronne-6, présente quant à lui une capacité d'adsorption de méthionine quasi nulle (8 % en moyenne de diminution des signaux RMN à 1 éq.).

### B.2/ Isotherme d'association avec un dérivé fluorescent de la méthionine en solution modèle à pH 3,5

Un dérivé fluorescent de la méthionine a été préparé en faisant réagir la *N*-BOC-homocystéine (préparée comme décrit dans la publication de Mejia, Polym. Chem., 2013, 4, 1969) avec la 7-allyloxocoumarine (préparée comme décrit dans la publication de Orhan, Bioorganic Chemistry, 84, 2019, 355). La *N*-boc-homocystéine (0,300 g, 1,27 mmol) a été dissoute dans du DMF sec (8 mL) et la solution purgée à l'argon pendant 10 min. La 7-allyloxocoumarine (0,257 g, 1,27 mmol) et de la 2,2,2-diméthoxyphényl-acétophénone (0,065 g, 0,255 mmol) ont été ajoutées et le mélange a été agité sous irradiation à 365 nm pendant 16 h. Le mélange a été dilué avec de l'acétate d'éthyle (20 mL) et lavé à l'eau (30 mL), à la saumure (20 mL), et ensuite séché sur Na₂SO₄, filtré et évaporé sous vide. Le solide ainsi obtenu a été purifié par chromatographie sur gel de silice (éluant: 3:1 EtOAc/hexane, puis EtOAc, et 9:1 EtOAc/MeOH) pour donner un solide jaune (176 mg, 31%) qui est déprotégé par exposition à une solution de HCl (4 M) dans le dioxane pour donner le produit souhaité (109 mg, 70%). ¹H RMN (300 MHz, 298 K, DMSO-*d*₆) δ 7.99 (d, *J* = 9.5 Hz, 1H, Hⱼ), 7.63 (d, *J* = 8.5 Hz, 1H, Hᵢ), 7.02 - 6.93 (m, 2H, H_{g} et Hₕ), 6.29 (d, *J* = 9.5 Hz, 1H, Hₖ), 4.16 (t, *J* = 6.2 Hz, 2H, H_{f}), 3.84 (t, *J =* 6.2 Hz, 1H, Hₐ), 2.74 - 2.59 (m, 4H, H_{c} et H_{d}), 2.10 - 1.94 (m, 4H, H_{b} and Hₑ)

¹³C RMN (75 MHz, 298 K, DMSO-*d*₆) δ 170.6, 161.7, 158.9, 155.4, 143.7, 130.5, 112.7, 112.5, 112.4, 100.2, 66.4, 52.30, 31.5, 28.6, 27.0, 26.5

ESI-MS (MeOH): 338.1047 (calc. pour C₁₆H₂₀NO₅S⁺ 338.1057), 360.0866 (calc. pour C₁₆H₁₉NO₅SNa⁺ 360.0876).

Le dosage de l'affinité du polymère envers la méthionine et la mesure de sa capacité d'extraction ont été réalisés comme suit. Une cellule en quartz de 1 cm de trajet optique a été remplie d'une solution d'eau / EtOH (12%) à pH 3 (2,0 mL) et de 0,254 mg de polymère PR1. Une solution concentrée de méthionine fluorescente (1 mM dans la même solution) a été ajoutée par aliquots de 25 µL. Après chaque ajout, la solution de la cellule a été mélangée pendant 16 h pour attendre l'équilibre et ensuite laissée décanter avant de déterminer la concentration de méthionine libre par absorption électronique à 324 nm (ε₃₂₄ = 10800 M⁻¹cm⁻¹). La courbe représentant l'absorbance mesurée en fonction de la concentration en méthionine ajoutée dans la cellule ainsi obtenue est montrée sur la figure 6.

La concentration de méthionine libre a ensuite été analysée par la technique de Scatchard en portant le rapport ([méthionine complexée] / [méthionine libre]) en fonction de la concentration en méthionine complexée. Par régression linéaire, on a trouvé une concentration en sites de complexation de 248 mg de méthionine par gramme de polymère PR1 (79 % de la quantité initiale de méthionine a été absorbée par le polymère. Cela correspond à 1,0 mg de polymère liant 0,298 mg de Met* à des concentrations voisines de 1.10⁻⁴ M, la concentration approximative de la méthionine dans le champagne). Deux sites de complexation distincts possédant des constantes d'association de 7,3 x 10⁶ M⁻¹ et 3,8 x 10⁵ M⁻¹ ont été observés. On en déduit une constante d'association moyenne pondérée par le nombre de sites égale à 3,0.10⁶ M⁻¹.

La même expérience, réalisée avec le polymère linéaire PL2, conduit à l'adsorption de 89 % de la méthionine fluorescente initiale par le polymère.

### C/ Protection en solution modèle soumise à irradiation - élimination des espèces soufrées volatiles

Une solution modèle de vin synthétique a été préparée en dissolvant 3,5 g d'acide tartrique dans 1 L d'un mélange eau 88% / éthanol 12% vol. et le pH a été ajusté à 3,5 en utilisant une solution aqueuse d'hydroxyde de sodium à 1M. Puis, la riboflavine (concentration en riboflavine de 0,05 mM) et la méthionine (0,1 mM) y ont été dissoutes. Cette solution a été agitée pendant 1 semaine à l'aide d'un agitateur orbitalaire en présence d'une quantité de 0,2 g/l de polymère réticulé conforme à l'invention PR1. Le matériau polymère a ensuite été filtré et la solution obtenue a été mise dans deux ampoules transparentes (2 x 4,5 ml) qui ont été scellées et irradiées pendant 5 min à 250 W/m². Les ampoules ont ensuite été ouvertes et la solution obtenue a été analysée par micro extraction sur phase solide couplée à la chromatographie en phase gazeuse avec détection par photométrie de flamme pulsée (SPME-GC-PFPD) en split 200, pour détecter les espèces suivantes : méthanethiol, diméthyldisulfure (DMDS) et diméthyltrisulfure (DMTS). En parallèle, un échantillon témoin sans résine a été traité de la même manière. Les résultats obtenus sont montrés dans le tableau 1.

**Tableau 1**

| | Sans polymère | Avec polymère PR1 |
|---|---|---|
| Méthanethiol (µg/l) | 184 | Non détecté |
| DMDS (µg/l) | 276 | 10,1 |
| DMTS (µg/l) | 133 | Non détecté |

Ces résultats indiquent clairement que le polymère conforme à l'invention a permis de diminuer de manière particulièrement importante les concentrations en espèces soufrées volatiles se formant dans la solution modèle consécutivement à l'exposition à la lumière : cette diminution est de 96 % pour le DMDS. Le méthanethiol et le DMTS se trouvent quant à eux chacun en quantité inférieure à son seuil de détection. Ces résultats démontrent que le matériau polymère a une capacité d'adsorption de méthionine suffisante pour prévenir quasiment totalement l'apparition des produits soufrés volatiles responsables du goût de lumière.

### D/ Traitement d'un vin effervescent

Le même protocole que celui décrit dans la section C/ ci-dessus a été appliqué à du vin effervescent répondant à l'appellation d'origine contrôlée Champagne, fraîchement ouvert, et en utilisant des quantités variables du matériau polymère PR1.

Sept échantillons ont été agités pendant 1 semaine : 5 échantillons destinés à être irradiés correspondent à des quantités de polymère variant de 0,2 g/l à 2 g/l, 1 échantillon correspond à un témoin sans polymère destiné à être irradié et 1 échantillon correspond à un témoin sans polymère qui ne sera pas irradié.

Les résultats obtenus sont montrés sur la figure 7, en termes de concentration totale en méthanethiol (MeSH) et diméthyldisulfure (DMDS) en fonction de la quantité de polymère utilisée. Ce vin effervescent après irradiation a par ailleurs été soumis à une analyse sensorielle par un panel sensoriel entrainé. La note de 4,2/10 lui a été attribuée sur le critère du goût de lumière. Le seuil de perception d'un goût de lumière par le panel sensoriel sur cet échantillon du vin irradié non en contact avec le polymère est illustré par une ligne en pointillé sur la figure. Comme on peut clairement le constater, le polymère conforme à l'invention a été efficace pour la prévention de l'apparition du MeSH et du DMDS dès les concentrations supérieures ou égales à 0,2 g/L. Au-dessus de cette concentration, la note attribuée par le panel sensoriel, sur le critère du goût de lumière, devrait ainsi être supérieure à 4,2/10.

Par rapport au témoin irradié sans polymère, pour l'échantillon irradié contenant 2 g/l de matériau polymère, on observe une diminution de 84% de la concentration en espèces soufrées volatiles MeSH et DMDS. Ceci démontre que suffisamment de méthionine a été séquestrée par le matériau polymère conforme à l'invention pour prévenir de manière quasi totale l'apparition des substances responsables du goût de lumière.

### E/ Compétitivité

Pour évaluer la sélectivité du polymère PR1 vis-à-vis de la méthionine par rapport à la proline, autre acide aminé présent en grande quantité dans les vins effervescents, des solutions contenant des concentrations égales de méthionine et proline (2 mM dans 0,5 ml de D₂O) ont été préparées. 0,4 mg de polymère PR1 ont ensuite été ajoutés à chaque solution et la diminution d'intensité des signaux RMN ¹H des deux acides aminés a été enregistrée. On obtient, pour la méthionine, une diminution moyenne des signaux de 41 %. La diminution moyenne des signaux relatifs à la proline est quant à elle largement moindre, égale à 19 %.

### F/ Recyclabilité

En prenant le contenu de la cuvette de l'expérience décrite au point B.2/ ci-dessus, il a été tenté d'éliminer la méthionine fluorescente Met* du polymère PR1 dans des conditions alcalines, afin que la capacité de liaison puisse être mesurée à nouveau de sorte à déterminer la recyclabilité du matériau. Le mode opératoire a été le suivant : le contenu de la cuvette (0,5 mg de polymère ayant atteint l'équilibre de reprise de Met*) a été transféré dans un tube à centrifuger, en rinçant la cuvette à l'eau (MilliQ, pH 10 par addition de NaOH). La suspension a été soumise aux ultrasons pendant 10 min, centrifugée (5000 tr/min, 8 min) et le surnageant a été soigneusement jeté sans rompre le culot. La résine a été lavée avec de l'eau (10 ml, MilliQ, pH 10 par addition de NaOH), soniquée, centrifugée et le surnageant jeté 5 fois de plus. Enfin, la résine a été rincée avec de l'eau (2 ml, pH = 3,0) pour s'assurer qu'il ne restait aucun résidu de NaOH, centrifugée et le surnageant a été jeté. La résine a été remise en suspension dans de l'eau (2,5 ml, pH = 3,0) et transférée dans une cuvette. Le spectre UV-visible a été enregistré. L'absorbance mesurée à 324 nm correspondait à une concentration de Met* de 7 % de la quantité initialement ajoutée.

A ce stade, Met* (0,5 ml, solution à 1 mM dans de l'eau à pH = 3,0) a été ajoutée (concentration de Met* = 1,67.10⁻⁴ M). Le mélange a été agité pendant une nuit pour lui permettre d'atteindre l'équilibre, et le spectre UV-visible a été enregistré à nouveau. L'absorbance mesurée à 324 nm correspondait à 45 % de la Met* qui a été ajoutée la deuxième fois, le restant ayant donc été lié par le polymère. Ceci démontre que le polymère conforme à l'invention peut être recyclé par lavages, ces derniers restaurant sa capacité d'adsorption de méthionine.

### G/ Stabilité pour contact alimentaire

Des études ont été réalisées pour quantifier la quantité de porphyrine et de cuivre libérées dans une solution modèle de vin (eau 88 %, éthanol 12 %, acide tartarique pour obtenir un pH de 3,5) après une mise en contact avec le polymère selon l'invention PR1 pendant 10 jours à 60 °C (conditions de vieillissement accéléré).

2,0 mg de polymère ont été mis en suspension dans 3,0 ml de la solution modèle et laissés au repos à la pièce température pendant une journée. La même expérience a été réalisée mais avec la solution chauffée à 60 °C pendant 10 jours. Les solutions ont été filtrées à travers un filtre seringue et les spectres UV-visible de chacune ont été enregistrés. Les porphyrines ont une très forte absorption à environ 420 nm et seraient facilement détectées si une espèce de porphyrine était dissoute. Pour chacune des expériences, aucun signal n'est détecté à 420 nm.

Pour détecter si des ions de cuivre s'échapperaient du matériau polymère dans les mêmes conditions, la solution de l'expérience ci-dessus laissée à 60 °C pendant 10 jours a été agitée vigoureusement pendant 10 min avec 5 ml d'une solution de DCM (7.10⁻⁷ M) de dithizone. La dithizone est un ligand organique qui absorbe fortement autour de 607 nm dans le spectre visible. Lors de la complexation des ions Cu(II), un nouveau maximum est observé vers 540 nm. Le spectre obtenu après exposition de la solution de dithizone à la solution aqueuse qui était en contact avec le polymère PR1 montre bien, par rapport au spectre de la dithizone seule, un nouveau maximum vers 540 nm, correspondant à la formation du complexe de la dithizone avec le Cu(II).

Il ressort de ce spectre qu'environ la moitié de la dithizone totale ajoutée a été complexée aux ions Cu(II). Ceci correspond à environ 0,01 % de la quantité totale d'ions Cu(II) supposément présente dans la masse de polymère PR1 utilisé : aucun relargage significatif de cuivre n'a ainsi été observé.

Il ressort de ces expériences que le matériau polymère PR1 selon l'invention n'a pas relargué ses composants porphyrine et ion cuivrique dans la solution modèle, et qu'il est compatible avec une utilisation au contact des aliments.

## Revendications

1. Procédé de séquestration de molécules de méthionine contenues dans un milieu liquide, comprenant la mise en contact dudit milieu liquide avec un matériau polymère solide comprenant des unités monomères à base de porphyrine contenant du cuivre.

2. Procédé selon la revendication 1, selon lequel ledit matériau polymère contient au moins un groupement hydrophile, de préférence un groupement ion sulfonate ou un de ses sels.

3. Procédé selon la revendication 1 ou 2, selon lequel au moins une desdites unités monomères répond à la formule générale (I) : 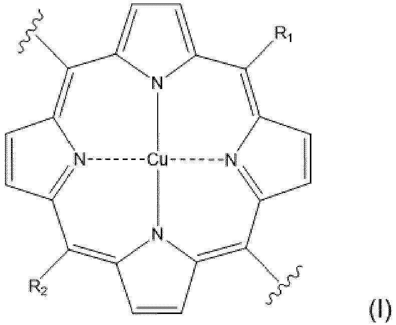 dans laquelle R₁ et R₂, identiques ou différents, représentent chacun un atome d'hydrogène ou un groupement de formule (II) : 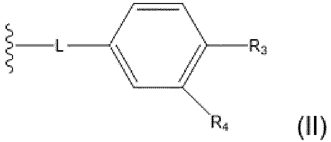 dans laquelle :
R₃ et R₄, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique,
et L représente une liaison covalente ou un bras espaceur.

4. Procédé selon la revendication 3, selon lequel, dans la formule (II), L représente un bras espaceur de formule (III) : 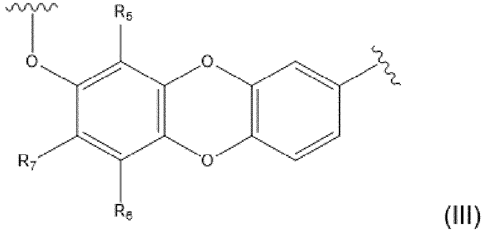 dans laquelle :
R₅ et R₆ représentent chacun un groupement carbonitrile -CN,
et R₇ représente un atome d'halogène, notamment un atome de fluor.

5. Procédé selon l'une quelconque des revendications 1 à 4, selon lequel lesdites unités monomères sont reliées par un groupe de liaison de formule (IV) : 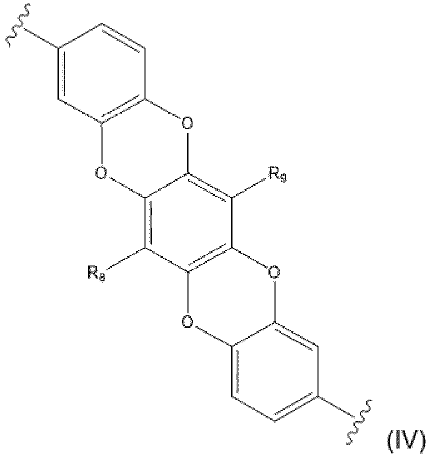 dans laquelle R₈ et R₉ représentent chacun un groupement carbonitrile -CN.

6. Procédé selon l'une quelconque des revendications 1 à 5, selon lequel ledit polymère est un polymère linéaire.

7. Procédé selon la revendication 6, selon lequel ledit polymère répond à la formule (V) : 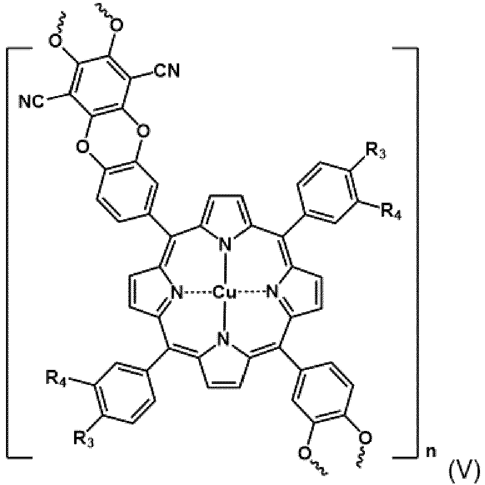 dans laquelle :
R₃ et R₄, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique,
et n est un nombre entier supérieur à 2.

8. Procédé selon la revendication 7, selon lequel, dans la formule (V), R₃ représente un atome d'hydrogène et R₄ représente un ion sulfonate ou un de ses sels.

9. Procédé selon l'une quelconque des revendications 1 à 5, selon lequel ledit polymère est un polymère réticulé.

10. Procédé selon la revendication 9, selon lequel ledit polymère est susceptible d'être obtenu par un procédé de synthèse comprenant des étapes de :
a/ préparation d'un dérivé de porphyrine de formule (VI) : 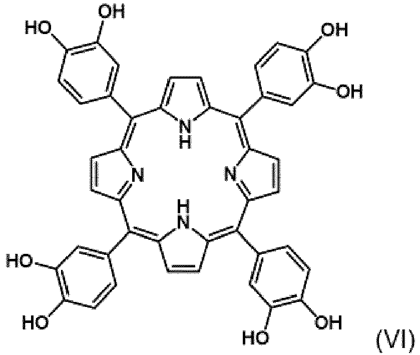
b/ polycondensation dudit dérivé de porphyrine de formule (VI) avec un composé de formule (VII) : 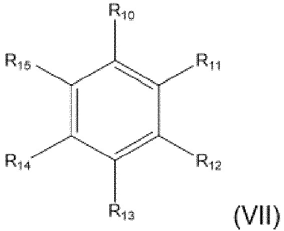 dans laquelle :
R₁₀ et R₁₃ représentent chacun un groupement carbonitrile -CN,
R₁₁, R₁₂, R₁₄ et R₁₅, identiques ou différents, représentent chacun un atome d'halogène, notamment un atome de fluor,
en présence d'un composé de formule (VIII) : 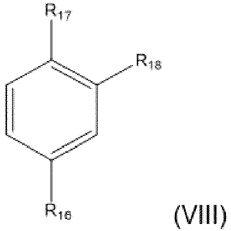 dans laquelle :
R₁₆ représente un groupement hydroxyle, amine primaire ou secondaire ou thiol, R₁₇ et R₁₈, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique, et
c/ complexation des noyaux porphyrine du polymère obtenu à l'étape b/ avec du cuivre.

11. Procédé selon la revendication 10, selon lequel, dans la formule (VIII), R₁₆ représente un groupement hydroxyle, R₁₇ représente un sel de sulfonate et R₁₈ représente un atome d'hydrogène.

12. Procédé selon l'une quelconque des revendications 1 à 11, selon lequel ledit matériau polymère est introduit dans ledit milieu liquide à une dose comprise entre 0,01 et 50 g/l.

13. Procédé selon l'une quelconque des revendications 1 à 12, comprenant une étape de séparation dudit milieu liquide et du matériau polymère séquestrant la méthionine.

14. Procédé selon la revendication 13, comprenant une étape finale de séparation des molécules de méthionine dudit matériau polymère.

15. Procédé de traitement d'un produit liquide alimentaire pour y diminuer le risque d'apparition de goût de lumière, **caractérisé en ce qu'**il comprend la mise en œuvre sur ledit produit liquide alimentaire d'un procédé de séquestration de molécules de méthionine contenues dans un milieu liquide selon l'une quelconque des revendications 1 à 14, la mise en contact dudit produit liquide alimentaire avec le matériau polymère étant réalisée pendant au moins 30 minutes, et étant suivie par une étape de séparation dudit matériau polymère et dudit produit liquide alimentaire.

16. Procédé de traitement d'un liquide alimentaire selon la revendication 15, selon lequel ledit produit liquide alimentaire est choisi parmi les vins, les produits laitiers et les produits brassicoles.

17. Procédé de détection de la présence de méthionine dans un liquide, **caractérisé en ce qu'**il comprend :
- la mise en œuvre, sur un échantillon dudit liquide, d'un procédé de séquestration de molécules de méthionine contenues dans un milieu liquide selon l'une quelconque des revendications 1 à 14, la mise en contact dudit échantillon de liquide avec le matériau polymère étant réalisée pendant au moins 30 minutes, et étant suivie par une étape de séparation dudit matériau polymère et dudit échantillon de liquide,
- puis l'analyse dudit matériau polymère pour la présence de méthionine.

18. Matériau polymère solide poreux susceptible d'être obtenu par un procédé de synthèse comprenant des étapes de :
a/ préparation d'un dérivé de porphyrine de formule (VI) : 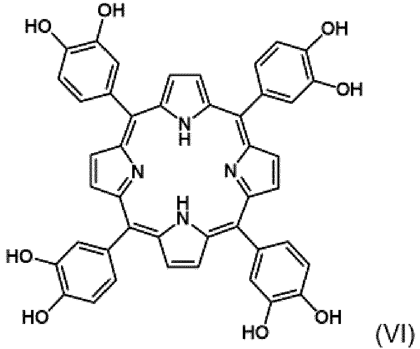
b/ polycondensation dudit dérivé de porphyrine de formule (VI) avec un composé de formule (VII) : 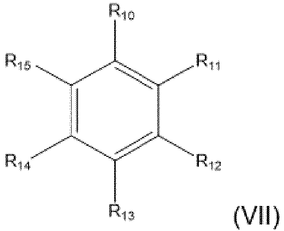 dans laquelle :
R₁₀ et R₁₃ représentent chacun un groupement carbonitrile -CN,
R₁₁, R₁₂, R₁₄ et R₁₅, identiques ou différents, représentent chacun un atome d'halogène, notamment un atome de fluor,
en présence d'un composé de formule (VIII) : 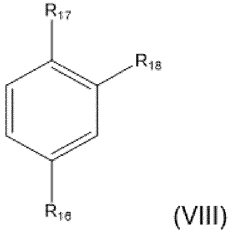 dans laquelle :
R₁₆ représente un groupement hydroxyle, amine primaire ou secondaire ou thiol, R₁₇ et R₁₈, identiques ou différents, représentent chacun un atome d'hydrogène ou un groupement choisi parmi un ion sulfonate ou un de ses sels, un ion carboxylate ou un de ses sels, un ion ammonium ou un de ses sels, ou un groupement acide boronique, et
c/ complexation des noyaux porphyrine du polymère obtenu à l'étape b/ avec du cuivre.

19. Matériau selon la revendication 18, dans lequel, dans la formule (VIII), R₁₆ représente un groupement hydroxyle, R₁₇ représente un sel de sulfonate, et R₁₈ représente un atome d'hydrogène.

## Patentansprüche

1. Verfahren zur Sequestrierung von Methioninmolekülen, die in einem flüssigen Medium enthalten sind, umfassend die Kontaktaufnahme des flüssigen Mediums mit einem festen Polymermaterial, das kupferhaltige Porphyrin-basierte Monomereinheiten umfasst.

2. Verfahren nach Anspruch 1, wobei das Polymermaterial mindestens eine hydrophile Gruppe, vorzugsweise eine Sulfonationengruppe oder eines ihrer Salze, enthält.

3. Verfahren nach Anspruch 1 oder 2, wobei mindestens eine der Monomereinheiten der allgemeinen Formel entspricht (I) : 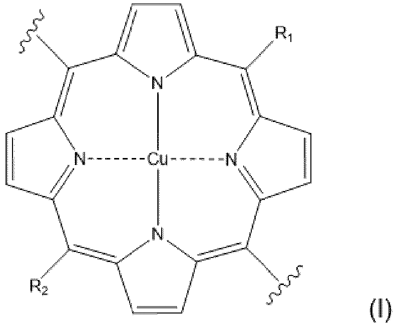 wobei R₁ und R₂, gleich oder verschieden, jeweils ein Wasserstoffatom oder eine Formelgruppe darstellen (II): 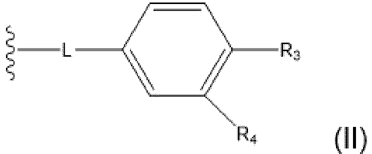 wobei:
R₃ und R₄, gleich oder verschieden, jeweils ein Wasserstoffatom oder eine Gruppe darstellen, ausgewählt aus einem Sulfonation oder einem seiner Salze, einem Carboxylation oder einem seiner Salze, einem Ammoniumion oder einem seiner Salze oder einer Borsäuregruppe,
und L eine kovalente Verbindung oder einen Abstandhalterarm darstellt.

4. Verfahren nach Anspruch 3, wobei, in der Formel (II), L einen Abstandhalterarm der Formel (III) darstellt: 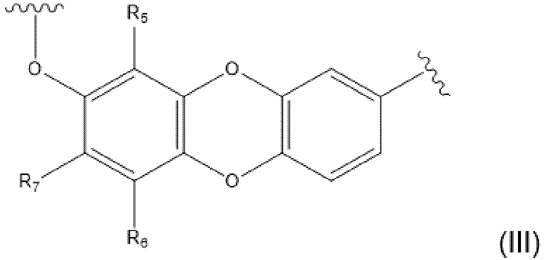 wobei:
R₅ und R₆ jeweils eine Carbonitril-CN-Gruppe darstellen,
und R₇ ein Halogenatom, insbesondere ein Fluoratom, darstellt.

5. Verfahren nach einem der Ansprüche 1 bis 4, wobei die Monomereinheiten durch eine Formelbindungsgruppe (IV) verbunden sind: 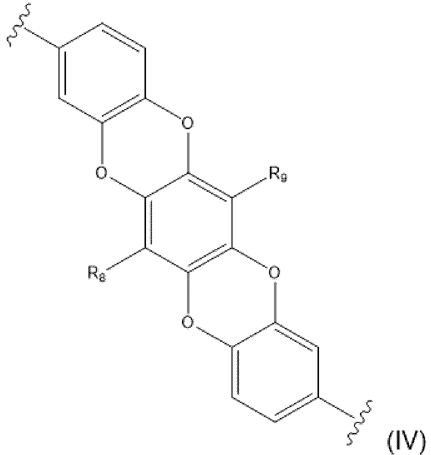 wobei R₈ und R₉ jeweils eine Carbonitril-CN-Gruppe darstellen.

6. Verfahren nach einem der Ansprüche 1 bis 5, wobei das Polymer ein lineares Polymer ist.

7. Verfahren nach Anspruch 6, wobei das Polymer der Formel (V) entspricht: 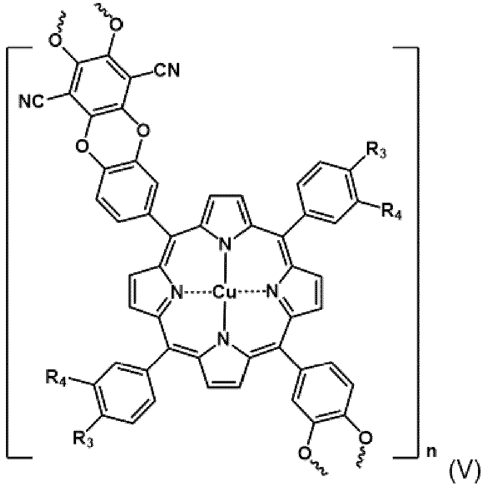 wobei:
R₃ und R₄, gleich oder verschieden, jeweils ein Wasserstoffatom oder eine Gruppe darstellen, ausgewählt aus einem Sulfonation oder einem seiner Salze, einem Carboxylation oder einem seiner Salze, einem Ammoniumion oder einem seiner Salze oder einer Borsäuregruppe,
und n eine ganze Zahl größer als 2 ist.

8. Verfahren nach Anspruch 7, wobei in der Formel (V), R₃ ein Wasserstoffatom darstellt und R₄ ein Sulfonation oder eines seiner Salze darstellt.

9. Verfahren nach einem der Ansprüche 1 bis 5, wobei das Polymer ein vernetztes Polymer ist.

10. Verfahren nach Anspruch 9, wobei das Polymer durch ein Syntheseverfahren erhalten werden kann, das Schritte umfasst zum:
a/ Herstellen eines Porphyrinderivats der Formel (VI): 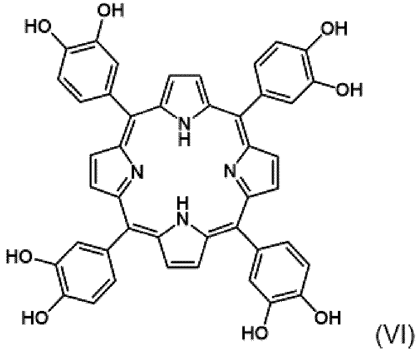
b/ Polykondensieren des Porphyrinderivats der Formel (VI) mit einer Verbindung der Formel (VII): 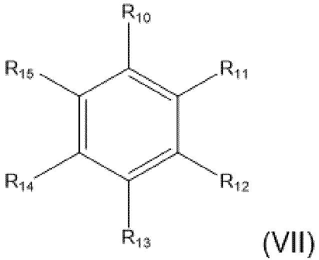 wobei:
R₁₀ und R₁₃ jeweils eine Carbonitril-CN-Gruppe darstellen, R₁₁, R₁₂, R₁₄ und R₁₅, gleich oder verschieden, jeweils ein Halogenatom, insbesondere ein Fluoratom, darstellen,
in Gegenwart einer Verbindung der Formel (VIII): 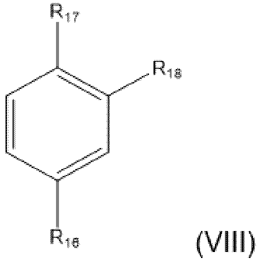 wobei:
R₁₆ eine Hydroxyl-, primäre oder sekundäre Amin- oder Thiolgruppe darstellt,
R₁₇ und R₁₈, gleich oder verschieden, jeweils ein Wasserstoffatom oder eine Gruppe darstellen, ausgewählt aus einem Sulfonation oder einem seiner Salze, einem Carboxylation oder einem seiner Salze, einem Ammoniumion oder einem seiner Salze oder einer Borsäuregruppe, und
c/ Komplexieren der Porphyrinkerne des in Schritt b/ erhaltenen Polymers mit Kupfer.

11. Verfahren nach Anspruch 10, wobei in der Formel (VIII), R₁₆ eine Hydroxylgruppe darstellt, R₁₇ ein Sulfonatsalz darstellt und R₁₈ ein Wasserstoffatom darstellt.

12. Verfahren nach einem der Ansprüche 1 bis 11, wobei das Polymermaterial in einer Dosis zwischen 0,01 und 50 g/l in das flüssige Medium eingeführt wird.

13. Verfahren nach einem der Ansprüche 1 bis 12, umfassend einen Schritt zum Trennen des flüssigen Mediums und des Methionin sequestrierenden Polymermaterials.

14. Verfahren nach Anspruch 13, umfassend einen letzten Schritt der Trennung der Methioninmoleküle aus dem Polymermaterial.

15. Verfahren zur Behandlung eines flüssigen Lebensmittelprodukts, um darin das Risiko des Auftretens von Lichtgeschmack zu verringern, **dadurch gekennzeichnet, dass** es die Durchführung eines Verfahrens zur Sequestrierung am flüssigen Lebensmittelprodukt von in einem flüssigen Medium enthaltenen Methioninmolekülen nach einem der Ansprüche 1 bis 14 umfasst, wobei die Kontaktaufnahme des flüssigen Lebensmittelprodukts mit dem Polymermaterial für mindestens 30 Minuten lang erfolgt, und darauf ein Schritt zur Trennung des Polymermaterials und des flüssigen Lebensmittelprodukts folgt.

16. Verfahren zur Verarbeitung einer Lebensmittelflüssigkeit nach Anspruch 15, wobei das flüssige Lebensmittelprodukt aus Weinen, Milchprodukten und Brauereiprodukten ausgewählt ist.

17. Verfahren zum Nachweis des Vorhandenseins von Methionin in einer Flüssigkeit, **dadurch gekennzeichnet, dass** es umfasst:
- Umsetzen eines Verfahrens zur Sequestrierung von Methioninmolekülen, die in einem flüssigen Medium enthalten sind, nach einem der Ansprüche 1 bis 14 , an einer Probe der Flüssigkeit, wobei die Kontaktaufnahme der Flüssigkeitsprobe mit dem Polymermaterial für mindestens 30 Minuten lang erfolgt und darauf ein Schritt zur Trennung des Polymermaterials und der Flüssigkeitsprobe folgt,
- danach Analysieren des Polymermaterials auf das Vorhandensein von Methionin.

18. Poröses festes Polymermaterial, das durch ein Syntheseverfahren erhalten werden kann, Schritte umfassend zum:
a/ Herstellen eines Porphyrinderivats der Formel (VI): 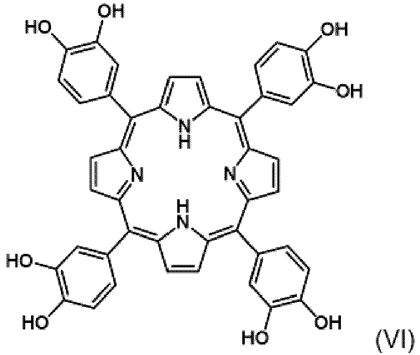
b/ Polykondensieren des Porphyrinderivats der Formel (VI) mit einer Verbindung der Formel (VII): 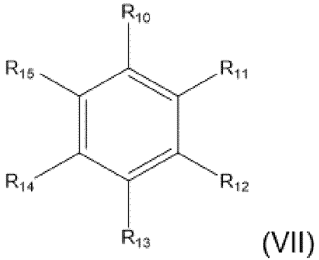 wobei:
R₁₀ und R₁₃ jeweils eine Carbonitril-CN-Gruppe darstellen, R₁₁, R₁₂, R₁₄ und R₁₅, gleich oder verschieden, jeweils ein Halogenatom, insbesondere ein Fluoratom, darstellen,
in Gegenwart einer Verbindung mit Formel (VIII): 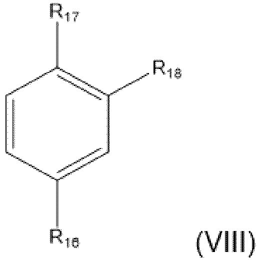 wobei:
R₁₆ eine Hydroxyl-, primäre oder sekundäre Amin- oder Thiolgruppe darstellt,
R₁₇ und R₁₈, gleich oder verschieden, jeweils ein Wasserstoffatom oder eine Gruppe darstellen, ausgewählt aus einem Sulfonation oder einem seiner Salze, einem Carboxylation oder einem seiner Salze, einem Ammoniumion oder einem seiner Salze oder einer Borsäuregruppe, und
c/ Komplexieren der Porphyrinkerne des in Schritt b/ erhaltenen Polymers mit Kupfer.

19. Material nach Anspruch 18, wobei, in der Formel (VIII), R₁₆ eine Hydroxylgruppe darstellt, R₁₇ ein Sulfonatsalz darstellt und R₁₈ ein Wasserstoffatom darstellt.

## Claims

1. Method for sequestering methionine molecules contained in a liquid medium, comprising contacting said liquid medium with a solid polymer material comprising monomer units based on porphyrin containing copper.

2. Method according to claim 1, wherein said polymer material contains at least one hydrophilic group, preferably a sulfonate ion group or a salt thereof.

3. Method according to claim 1 or 2, wherein at least one of said monomer units has the general formula (I): 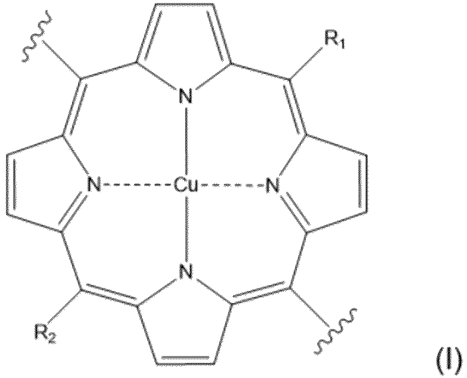 wherein R₁ and R₂, identical or different, each represent a hydrogen atom or a group of formula (II): 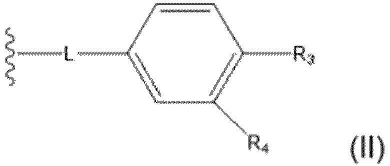 wherein:
R₃ and R₄, identical or different, each represent a hydrogen atom or a group selected from among a sulfonate ion or a salt thereof, a carboxylate ion or a salt thereof, an ammonium ion or a salt thereof, or a boronic acid group, and L represents a covalent bond or a spacer arm.

4. Method according to claim 3, wherein, in formula (II), L represents a spacer arm of formula (III): 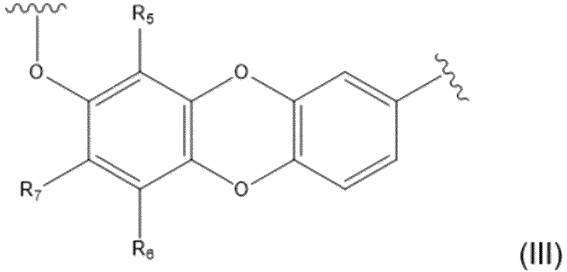 wherein:
R₅ and R₆ each represent a carbonitrile group -CN,
and R₇ represents a halogen atom, in particular a fluorine atom.

5. Method according to any one of claims 1 to 4, wherein said monomer units are connected by a bonding group of formula (IV): 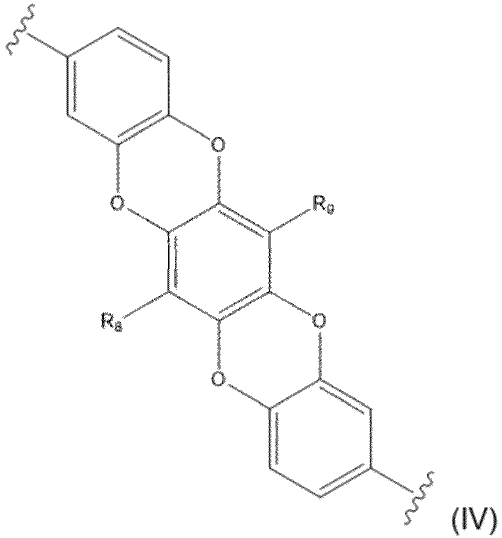 wherein R₈ and R₉ each represent a carbonitrile group -CN.

6. Method according to any one of claims 1 to 5, wherein said polymer is a linear polymer.

7. Method according to claim 6, wherein said polymer has the formula (V): 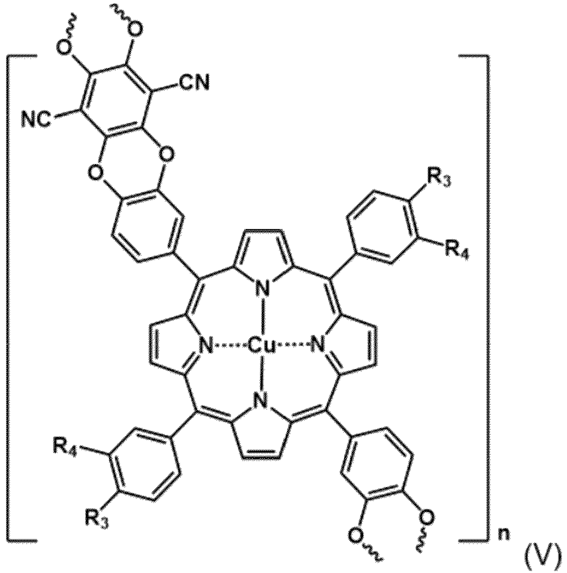 wherein:
R₃ and R₄, identical or different, each represent a hydrogen atom or a group selected from among a sulfonate ion or a salt thereof, a carboxylate ion or a salt thereof, an ammonium ion or a salt thereof, or a boronic acid group, and n is an integer greater than 2.

8. Method according to claim 7, wherein, in formula (V), R₃ represents a hydrogen atom and R₄ represents a sulfonate ion or a salt thereof.

9. Method according to any one of claims 1 to 5, wherein said polymer is a crosslinked polymer.

10. Method according to claim 9, wherein said polymer is obtainable by a synthesis method comprising the steps of:
a/ preparing a porphyrin derivative of formula (VI): 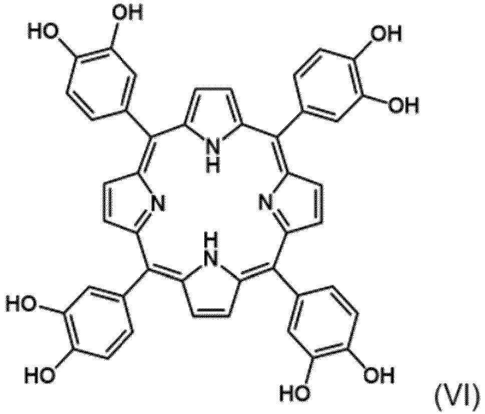
b/ polycondensing said porphyrin derivative of formula (VI) with a compound of formula (VII): 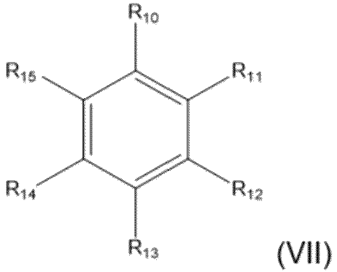 wherein:
R₁₀ and R₁₃ each represent a carbonitrile group -CN,
R₁₁, R₁₂, R₁₄ and R₁₅, identical or different, each represent a halogen atom, in particular a fluorine atom,
in the presence of a compound of formula (VIII): 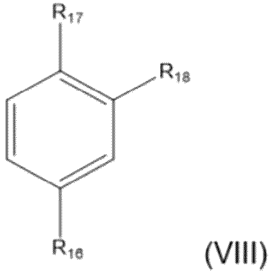 wherein:
R₁₆ represents a hydroxyl, primary or secondary amine or thiol group,
R₁₇ and R₁₈, identical or different, each represent a hydrogen atom or a group selected from among a sulfonate ion or a salt thereof, a carboxylate ion or a salt thereof, an ammonium ion or a salt thereof, or a boronic acid group, and
c/ complexing the porphyrin rings of the polymer obtained in step b/ with copper.

11. Method according to claim 10, wherein, in formula (VIII), R₁₆ represents a hydroxyl group, R₁₇ represents a sulfonate salt and R₁₈ represents a hydrogen atom.

12. Method according to any one of claims 1 to 11, wherein said polymer material is introduced into said liquid medium at a dose comprised between 0.01 and 50 g/l.

13. Method according to any one of claims 1 to 12, comprising a step of separating said liquid medium and the polymer material sequestering methionine.

14. Method according to claim 13, comprising a final step of separating the methionine molecules from said polymer material.

15. Method of treating a liquid food product to reduce the risk of a light-struck taste occurring therein, **characterized in that** it comprises implementing on said liquid food product a method for sequestering methionine molecules contained in a liquid medium according to any one of claims 1 to 14, the contacting of said liquid food product with the polymer material being carried out for at least 30 minutes, and being followed by a step of separating said polymer material and said liquid food product.

16. Method of treating a food liquid according to claim 15, wherein said liquid food product is selected from among wines, dairy products and malting products.

17. Method of detecting the presence of methionine in a liquid, **characterized in that** it comprises:
- implementing, on a sample of said liquid, a method for sequestering methionine molecules contained in a liquid medium according to any one of claims 1 to 14, the contacting of said liquid sample with the polymer material being carried out for at least 30 minutes, and being followed by a step of separating said polymer material and said liquid sample,
- then analyzing said polymer material for the presence of methionine.

18. Porous solid polymer material obtainable by a synthesis method comprising the steps of:
a/ preparing a porphyrin derivative of formula (VI): 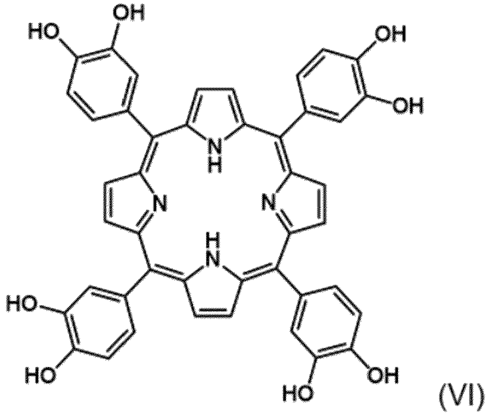
b/ polycondensing said porphyrin derivative of formula (VI) with a compound of formula (VII): 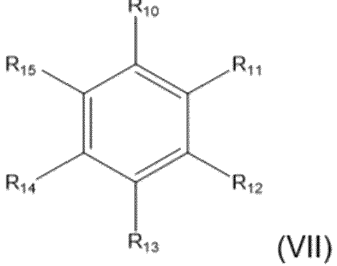 wherein:
R₁₀ and R₁₃ each represent a carbonitrile group -CN,
R₁₁, R₁₂, R₁₄ and R₁₅, identical or different, each represent a halogen atom, in particular a fluorine atom,
in the presence of a compound of formula (VIII): 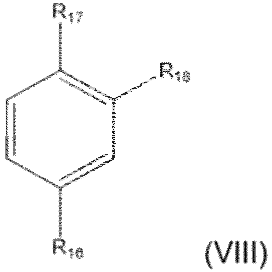 wherein:
R₁₆ represents a hydroxyl, primary or secondary amine or thiol group,
R₁₇ and R₁₈, identical or different, each represent a hydrogen atom or a group selected from among a sulfonate ion or a salt thereof, a carboxylate ion or a salt thereof, an ammonium ion or a salt thereof, or a boronic acid group,
and
c/ complexing the porphyrin rings of the polymer obtained in step b/ with copper.

19. Material according to claim 18, wherein, in formula (VIII), R₁₆ represents a hydroxyl group, R₁₇ represents a sulfonate salt, and R₁₈ represents a hydrogen atom.
